# EUROPEAN PATENT APPLICATION

(11) **EP 2 009 026 A1**
(43) Date of publication of application: **31.12.2008**
(21) Application number: 07012752.7
(22) Date of filing: 29.06.2007
(51) Int. Cl.: C07K 16/28, C07K 16/46, C12N 15/13, C12N 5/20, A61K 39/395, A61P 7/00

(54) **Anti-VPAC1 antibodies and their uses**

(71) Applicant: ThromboGenics N.V., 3000 Leuven (BE); Life Sciences Research Partners, 3000 Leuven (BE)
(72) Inventor: Freson, Kathleen, 3730 Hoeselt (BE); van Geet, Chris, 3221 Nieuwrode (BE); Hoylaerts, Marc, 3010 Kessel-Lo (BE); Stassen, Jean-Marie, 3210 Lubbeek (BE)
(74) Representative: Bird, Ariane

(57) **Abstract**

Monoclonal antibodies, in particular monoclonal antibodies to the VPAC1 receptor protein, compositions containing them and nucleic acid sequences encoding them. Host cells expressing said monoclonal antibodies, recombinant (expression) vectors and methods for producing said antibodies. Prevention or treatment of thrombocytopenia with antibodies to the VPAC1 receptor.

SEQUENCE LISTING

<110> ThromboGenics N.V.
D. Collen Research Foundation vzw

<120> Anti-VPAC1 antibodies and their uses

<130> C4390-EP

<160> 15

<170> PatentIn version 3.3

<210> 1
<211> 18
<212> PRT
<213> human

<400> 1

Ile His Phe Glu Asp Tyr Gly Cys Trp Asp Thr Ile Asn Ser Ser Leu
1               5                   10                  15

Trp Trp

<210> 2
<211> 13
<212> PRT
<213> human

<400> 2

Pro Asp Asn Phe Lys Pro Glu Val Lys Met Leu Phe Glu
1               5                   10

<210> 3
<211> 8
<212> PRT
<213> mouse

<400> 3

Gly Asp Ser Ile Thr Ser Gly Tyr
1               5

<210> 4
<211> 7
<212> PRT
<213> mouse

<400> 4

Ile Arg Tyr Ser Gly Ser Thr
1               5

<210> 5
<211> 11
<212> PRT
<213> mouse

<400> 5

Ser Asn Trp Ala Tyr Trp Gly Gln Gly Thr Leu
1               5                   10

<210>  6
<211>  11
<212>  PRT
<213>  mouse

<400>  6

Lys Ser Leu Leu Tyr Lys Asp Gly Lys Thr Tyr
1               5                   10

<210>  7
<211>  3
<212>  PRT
<213>  mouse

<400>  7

Leu Met Ser
1

<210>  8
<211>  9
<212>  PRT
<213>  mouse

<400>  8

Gln Gln Leu Val Glu Tyr Pro Leu Thr
1               5

<210>  9
<211>  111
<212>  PRT
<213>  mouse

<400>  9

Glu Val Gln Leu Gln Glu Ser Gly Pro Ser Leu Val Lys Pro Ser Gln
1               5                   10                  15

Thr Leu Ser Leu Thr Cys Ser Val Thr Gly Asp Ser Ile Thr Ser Gly
            20                  25                  30

Tyr Trp Asn Trp Ile Arg Lys Phe Pro Gly Asn Lys Leu Glu Tyr Met
        35                  40                  45

Gly Tyr Ile Arg Tyr Ser Gly Ser Thr Tyr Tyr Ser Pro Ala Leu Lys
    50                  55                  60

Ser Arg Phe Ser Ile Thr Arg Asp Thr Ser Lys Asn Gln Tyr Tyr Leu
65                  70                  75                  80

Gln Leu Asn Ser Val Thr Thr Glu Asp Thr Ala Thr Tyr Tyr Cys Ser
                85                  90                  95

Asn Trp Ala Tyr Trp Gly Gln Gly Thr Leu Val Thr Val Ser Ala
            100                 105                 110

<210> 10
<211> 112
<212> PRT
<213> mouse

<400> 10

Asp Ile Val Ile Thr Gln Asp Glu Leu Ser Asn Pro Val Thr Ser Gly
1               5                   10                  15

Glu Ser Val Ser Ile Ser Cys Arg Ser Ser Lys Ser Leu Leu Tyr Lys
            20                  25                  30

Asp Gly Lys Thr Tyr Leu Asn Trp Phe Leu Gln Arg Pro Gly Gln Ser
        35                  40                  45

Pro Gln Leu Leu Ile Tyr Leu Met Ser Thr Arg Ala Ser Gly Val Ser
    50                  55                  60

Asp Arg Phe Ser Gly Ser Gly Ser Gly Thr Asp Phe Thr Leu Glu Ile
65                  70                  75                  80

Ser Arg Val Lys Ala Glu Asp Val Gly Val Tyr Tyr Cys Gln Gln Leu
                85                  90                  95

Val Glu Tyr Pro Leu Thr Phe Gly Ala Gly Thr Lys Leu Glu Leu Lys
            100                 105                 110

<210> 11
<211> 460
<212> PRT
<213> artificial sequence

<220>
<223> humanized IgG1 antibody / heavy chain

<400> 11

Met Asp Trp Thr Trp Arg Ile Leu Phe Leu Val Ala Ala Ala Thr Gly
1               5                   10                  15

Ala His Ser Glu Val Gln Leu Gln Glu Ser Gly Pro Gly Leu Val Lys
            20                  25                  30

Pro Ser Gln Thr Leu Ser Leu Thr Cys Thr Val Ser Gly Asp Ser Ile
        35                  40                  45

Thr Ser Gly Tyr Trp Asn Trp Ile Arg Lys His Pro Gly Lys Gly Leu
    50                  55                  60

Glu Tyr Met Gly Tyr Ile Arg Tyr Ser Gly Ser Thr Tyr Tyr Ser Pro
65                  70                  75                  80

Ser Leu Lys Ser Arg Phe Thr Ile Ser Arg Asp Thr Ser Lys Asn Gln
                85                  90                  95

Tyr Ser Leu Lys Leu Ser Ser Val Thr Ala Ala Asp Thr Ala Thr Tyr
            100                 105                 110

Tyr Cys Ser Asn Trp Ala Tyr Trp Gly Gln Gly Thr Leu Val Thr Val
        115                 120                 125

Ser Ala Ala Ser Thr Lys Gly Pro Ser Val Phe Pro Leu Ala Pro Ser
    130                 135                 140

Ser Lys Ser Thr Ser Gly Gly Thr Ala Ala Leu Gly Cys Leu Val Lys
145                 150                 155                 160

Asp Tyr Phe Pro Glu Pro Val Thr Val Ser Trp Asn Ser Gly Ala Leu
                165                 170                 175

Thr Ser Gly Val His Thr Phe Pro Ala Val Leu Gln Ser Ser Gly Leu
            180                 185                 190

Tyr Ser Leu Ser Ser Val Val Thr Val Pro Ser Ser Ser Leu Gly Thr
        195                 200                 205

Gln Thr Tyr Ile Cys Asn Val Asn His Lys Pro Ser Asn Thr Lys Val
    210                 215                 220

Asp Lys Arg Val Glu Pro Lys Ser Cys Asp Lys Thr His Thr Cys Pro
225                 230                 235                 240

Pro Cys Pro Ala Pro Glu Leu Leu Gly Gly Pro Ser Val Phe Leu Phe
                245                 250                 255

Pro Pro Lys Pro Lys Asp Thr Leu Met Ile Ser Arg Thr Pro Glu Val
            260                 265                 270

Thr Cys Val Val Val Asp Val Ser His Glu Asp Pro Glu Val Lys Phe
        275                 280                 285

Asn Trp Tyr Val Asp Gly Val Glu Val His Asn Ala Lys Thr Lys Pro
    290                 295                 300

Arg Glu Glu Gln Tyr Asn Ser Thr Tyr Arg Val Val Ser Val Leu Thr
305                 310                 315                 320

Val Leu His Gln Asp Trp Leu Asn Gly Lys Glu Tyr Lys Cys Lys Val
                325                 330                 335

Ser Asn Lys Ala Leu Pro Ala Pro Ile Glu Lys Thr Ile Ser Lys Ala
            340                 345                 350

Lys Gly Gln Pro Arg Glu Pro Gln Val Tyr Thr Leu Pro Pro Ser Arg
        355                 360                 365

Glu Glu Met Thr Lys Asn Gln Val Ser Leu Thr Cys Leu Val Lys Gly
    370                 375                 380

Phe Tyr Pro Ser Asp Ile Ala Val Glu Trp Glu Ser Asn Gly Gln Pro
385                 390                 395                 400

Glu Asn Asn Tyr Lys Thr Thr Pro Pro Val Leu Asp Ser Asp Gly Ser
                405                 410                 415

Phe Phe Leu Tyr Ser Lys Leu Thr Val Asp Lys Ser Arg Trp Gln Gln
            420                 425                 430

Gly Asn Val Phe Ser Cys Ser Val Met His Glu Ala Leu His Asn His
        435                 440                 445

Tyr Thr Gln Lys Ser Leu Ser Leu Ser Pro Gly Lys
    450                 455                 460

<210>  12
<211>  237
<212>  PRT
<213>  artificial sequence

<220>
<223>  humanized IgG1 antibody / light chain

<400>  12

Met Glu Thr Pro Ala Gln Leu Leu Phe Leu Leu Leu Leu Trp Leu Pro
1               5                   10                  15

Asp Thr Thr Gly Asp Ile Val Ile Thr Gln Ser Pro Leu Ser Leu Pro
            20                  25                  30

Val Thr Pro Gly Glu Pro Val Ser Ile Ser Cys Arg Ser Ser Lys Ser
        35                  40                  45

Leu Leu Tyr Lys Asp Gly Lys Thr Tyr Leu Asn Trp Phe Leu Gln Arg
    50                  55                  60

Pro Gly Gln Ser Pro Gln Leu Leu Ile Tyr Leu Met Ser Thr Arg Ala
65                  70                  75                  80

Ser Gly Val Ser Asp Arg Phe Ser Gly Ser Gly Ser Gly Thr Asp Phe
                85                  90                  95

Thr Leu Lys Ile Ser Arg Val Glu Ala Glu Asp Val Gly Val Tyr Tyr
            100                 105                 110

Cys Gln Gln Leu Val Glu Tyr Pro Leu Thr Phe Gly Gln Gly Thr Lys

115                 120                 125

Leu Glu Ile Lys Gln Pro Lys Ala Ala Pro Ser Val Thr Leu Phe Pro
    130                 135                 140

Pro Ser Ser Glu Glu Leu Gln Ala Asn Lys Ala Thr Leu Val Cys Leu
145                 150                 155                 160

Ile Ser Asp Phe Tyr Pro Gly Ala Val Thr Val Ala Trp Lys Ala Asp
                165                 170                 175

Ser Ser Pro Val Lys Ala Gly Val Glu Thr Thr Thr Pro Ser Lys Gln
            180                 185                 190

Ser Asn Asn Lys Tyr Ala Ala Ser Ser Tyr Leu Ser Leu Thr Pro Glu
        195                 200                 205

Gln Trp Lys Ser His Arg Ser Tyr Ser Cys Gln Val Thr His Glu Gly
    210                 215                 220

Ser Thr Val Glu Lys Thr Val Ala Pro Thr Glu Cys Ser
225                 230                 235

<210>  13
<211>  457
<212>  PRT
<213>  artificial sequence

<220>
<223>  humanized IgG4 antibody / heavy chain

<400>  13

Met Asp Trp Thr Trp Arg Ile Leu Phe Leu Val Ala Ala Ala Thr Gly
1               5                   10                  15

Ala His Ser Glu Val Gln Leu Gln Glu Ser Gly Pro Gly Leu Val Lys
            20                  25                  30

Pro Ser Gln Thr Leu Ser Leu Thr Cys Thr Val Ser Gly Asp Ser Ile
        35                  40                  45

Thr Ser Gly Tyr Trp Asn Trp Ile Arg Lys His Pro Gly Lys Gly Leu
    50                  55                  60

Glu Tyr Met Gly Tyr Ile Arg Tyr Ser Gly Ser Thr Tyr Tyr Ser Pro
65                  70                  75                  80

Ser Leu Lys Ser Arg Phe Thr Ile Ser Arg Asp Thr Ser Lys Asn Gln
                85                  90                  95

Tyr Ser Leu Lys Leu Ser Ser Val Thr Ala Ala Asp Thr Ala Thr Tyr
            100                 105                 110

Tyr Cys Ser Asn Trp Ala Tyr Trp Gly Gln Gly Thr Leu Val Thr Val
        115                 120                 125

Ser Ala Ala Ser Thr Lys Gly Pro Ser Val Phe Pro Leu Ala Pro Cys
    130                 135                 140

Ser Arg Ser Thr Ser Glu Ser Thr Ala Ala Leu Gly Cys Leu Val Lys
145                 150                 155                 160

Asp Tyr Phe Pro Glu Pro Val Thr Val Ser Trp Asn Ser Gly Ala Leu
                165                 170                 175

Thr Ser Gly Val His Thr Phe Pro Ala Val Leu Gln Ser Ser Gly Leu
            180                 185                 190

Tyr Ser Leu Ser Ser Val Val Thr Val Pro Ser Ser Ser Leu Gly Thr
        195                 200                 205

Lys Thr Tyr Thr Cys Asn Val Asp His Lys Pro Ser Asn Thr Lys Val
    210                 215                 220

Asp Lys Arg Val Glu Ser Lys Tyr Gly Pro Pro Cys Pro Ser Cys Pro
225                 230                 235                 240

Ala Pro Glu Phe Leu Gly Gly Pro Ser Val Phe Leu Phe Pro Pro Lys
                245                 250                 255

Pro Lys Asp Thr Leu Met Ile Ser Arg Thr Pro Glu Val Thr Cys Val
            260                 265                 270

Val Val Asp Val Ser Gln Glu Asp Pro Glu Val Gln Phe Asn Trp Tyr
        275                 280                 285

Val Asp Gly Val Glu Val His Asn Ala Lys Thr Lys Pro Arg Glu Glu
    290                 295                 300

Gln Phe Asn Ser Thr Tyr Arg Val Val Ser Val Leu Thr Val Val His
305                 310                 315                 320

Gln Asp Trp Leu Asn Gly Lys Glu Tyr Lys Cys Lys Val Ser Asn Lys
                325                 330                 335

Gly Leu Pro Ser Ser Ile Glu Lys Thr Ile Ser Lys Ala Lys Gly Gln
            340                 345                 350

Pro Arg Glu Pro Gln Val Tyr Thr Leu Pro Pro Ser Gln Glu Glu Met
        355                 360                 365

Thr Lys Asn Gln Val Ser Leu Thr Cys Leu Val Lys Gly Phe Tyr Pro
    370                 375                 380

Ser Asp Ile Ala Val Glu Trp Glu Ser Asn Gly Gln Pro Glu Asn Asn
385                 390                 395                 400

Tyr Lys Thr Thr Pro Pro Val Leu Asp Ser Asp Gly Ser Phe Phe Leu
                405                 410                 415

Tyr Ser Arg Leu Thr Val Asp Lys Ser Arg Trp Gln Glu Gly Asn Val
            420                 425                 430

Phe Ser Cys Ser Val Met His Glu Ala Leu His Asn His Tyr Thr Gln
        435                 440                 445

Lys Ser Leu Ser Leu Ser Leu Gly Lys
    450                 455

<210>  14
<211>  239
<212>  PRT
<213>  artificial sequence

<220>
<223>  humanized IgG4 antibody / light chain

<400>  14

Met Glu Thr Pro Ala Gln Leu Leu Phe Leu Leu Leu Leu Trp Leu Pro
1               5                   10                  15

Asp Thr Thr Gly Asp Ile Val Ile Thr Gln Ser Pro Leu Ser Leu Pro
            20                  25                  30

Val Thr Pro Gly Glu Pro Val Ser Ile Ser Cys Arg Ser Ser Lys Ser
        35                  40                  45

Leu Leu Tyr Lys Asp Gly Lys Thr Tyr Leu Asn Trp Phe Leu Gln Arg
    50                  55                  60

Pro Gly Gln Ser Pro Gln Leu Leu Ile Tyr Leu Met Ser Thr Arg Ala
65                  70                  75                  80

Ser Gly Val Ser Asp Arg Phe Ser Gly Ser Gly Ser Gly Thr Asp Phe
                85                  90                  95

Thr Leu Lys Ile Ser Arg Val Glu Ala Glu Asp Val Gly Val Tyr Tyr
            100                 105                 110

Cys Gln Gln Leu Val Glu Tyr Pro Leu Thr Phe Gly Gln Gly Thr Lys
        115                 120                 125

Leu Glu Ile Lys Arg Thr Val Ala Ala Pro Ser Val Phe Ile Phe Pro
    130                 135                 140

Pro Ser Asp Glu Gln Leu Lys Ser Gly Thr Ala Ser Val Val Cys Leu

145                 150                 155                 160

Leu Asn Asn Phe Tyr Pro Arg Glu Ala Lys Val Gln Trp Lys Val Asp
                165                 170                 175

Asn Ala Leu Gln Ser Gly Asn Ser Gln Glu Ser Val Thr Glu Gln Asp
            180                 185                 190

Ser Lys Asp Ser Thr Tyr Ser Leu Ser Ser Thr Leu Thr Leu Ser Lys
        195                 200                 205

Ala Asp Tyr Glu Lys His Lys Leu Tyr Ala Cys Glu Val Thr His Gln
    210                 215                 220

Gly Leu Ser Ser Pro Val Thr Lys Ser Phe Asn Arg Gly Glu Cys
225                 230                 235

<210>  15
<211>  457
<212>  PRT
<213>  human

<400>  15

Met Arg Pro Pro Ser Pro Leu Pro Ala Arg Trp Leu Cys Val Leu Ala
1               5                   10                  15

Gly Ala Leu Ala Trp Ala Leu Gly Pro Ala Gly Gly Gln Ala Ala Arg
            20                  25                  30

Leu Gln Glu Glu Cys Asp Tyr Val Gln Met Ile Glu Val Gln His Lys
        35                  40                  45

Gln Cys Leu Glu Glu Ala Gln Leu Glu Asn Glu Thr Ile Gly Cys Ser
    50                  55                  60

Lys Met Trp Asp Asn Leu Thr Cys Trp Pro Ala Thr Pro Arg Gly Gln
65                  70                  75                  80

Val Val Val Leu Ala Cys Pro Leu Ile Phe Lys Leu Phe Ser Ser Ile
                85                  90                  95

Gln Gly Arg Asn Val Ser Arg Ser Cys Thr Asp Glu Gly Trp Thr His
            100                 105                 110

Leu Glu Pro Gly Pro Tyr Pro Ile Ala Cys Gly Leu Asp Asp Lys Ala
        115                 120                 125

Ala Ser Leu Asp Glu Gln Gln Thr Met Phe Tyr Gly Ser Val Lys Thr
    130                 135                 140

Gly Tyr Thr Ile Gly Tyr Gly Leu Ser Leu Ala Thr Leu Leu Val Ala
145                 150                 155                 160

Thr Ala Ile Leu Ser Leu Phe Arg Lys Leu His Cys Thr Arg Asn Tyr
                165                 170                 175

Ile His Met His Leu Phe Ile Ser Phe Ile Leu Arg Ala Ala Ala Val
            180                 185                 190

Phe Ile Lys Asp Leu Ala Leu Phe Asp Ser Gly Glu Ser Asp Gln Cys
        195                 200                 205

Ser Glu Gly Ser Val Gly Cys Lys Ala Ala Met Val Phe Phe Gln Tyr
    210                 215                 220

Cys Val Met Ala Asn Phe Phe Trp Leu Leu Val Glu Gly Leu Tyr Leu
225                 230                 235                 240

Tyr Thr Leu Leu Ala Val Ser Phe Phe Ser Glu Arg Lys Tyr Phe Trp
                245                 250                 255

Gly Tyr Ile Leu Ile Gly Trp Gly Val Pro Ser Thr Phe Thr Met Val
            260                 265                 270

Trp Thr Ile Ala Arg Ile His Phe Glu Asp Tyr Gly Cys Trp Asp Thr
        275                 280                 285

Ile Asn Ser Ser Leu Trp Trp Ile Ile Lys Gly Pro Ile Leu Thr Ser
    290                 295                 300

Ile Leu Val Asn Phe Ile Leu Phe Ile Cys Ile Ile Arg Ile Leu Leu
305                 310                 315                 320

Gln Lys Leu Arg Pro Pro Asp Ile Arg Lys Ser Asp Ser Ser Pro Tyr
                325                 330                 335

Ser Arg Leu Ala Arg Ser Thr Leu Leu Leu Ile Pro Leu Phe Gly Val
            340                 345                 350

His Tyr Ile Met Phe Ala Phe Phe Pro Asp Asn Phe Lys Pro Glu Val
        355                 360                 365

Lys Met Val Phe Glu Leu Val Val Gly Ser Phe Gln Gly Phe Val Val
    370                 375                 380

Ala Ile Leu Tyr Cys Phe Leu Asn Gly Glu Val Gln Ala Glu Leu Arg
385                 390                 395                 400

Arg Lys Trp Arg Arg Trp His Leu Gln Gly Val Leu Gly Trp Asn Pro
                405                 410                 415

Lys Tyr Arg His Pro Ser Gly Gly Ser Asn Gly Ala Thr Cys Ser Thr
            420                 425                 430

Gln Val Ser Met Leu Thr Arg Val Ser Pro Gly Ala Arg Arg Ser Ser
        435                 440                 445

Ser Phe Gln Ala Glu Val Ser Leu Val
    450                 455

## Description

### FIELD OF THE INVENTION

The invention relates to monoclonal antibodies, in particular monoclonal antibodies to the VPAC1 receptor protein, compositions containing them and nucleic acid sequences encoding them. The invention also relates to host cells expressing said monoclonal antibodies, to recombinant (expression) vectors and to methods for producing said antibodies. The invention further relates to the field of thrombocytopenia, and in particular to the prevention or treatment of thrombocytopenia with antibodies to the VPAC1 receptor.

### BACKGROUND OF THE INVENTION

Thrombocytopenia is the medical term for any condition in which the number of platelets in the blood is lower than normal. Thrombocytopenia may be the result of a variety of causes including immune disorders and genetic defects, or may be induced by a number of medicinal treatments including chemotherapy. Treatment of thrombocytopenia may include medications that block the antibodies that attack platelets, such as corticosteroids, or medications that suppress the immune system to reduce antibody formation, such as cyclophosphamide (Cytoxan) or azathioprine (Imuran). These are used in treating, e.g., idiopathic thrombocytopenic purpura (ITP). If chronic ITP doesn't respond to corticosteroids, surgical removal of the spleen (splenectomy) may help. Blood transfusions can replace lost blood (and the platelets contained therein). Severe thrombocytopenia, such as related to cancer or chemotherapy, is usually treated by administering platelet concentrates. Each year in the United States, approximately eight million units of platelets are transfused into patients to reduce the risk of severe bleeding (Kaushansky, 1998). It should be noted, however, that at least 30% of such transfusions result in complications (Kaushansky 1998). Thrombopoetin (TPO) had limited success for the treatment of thrombocytopenia, primarily due to antibody production. The second generation of TPO like agents has been developed are called TPO mimetics and should not trigger auto-immune anti-TPO antibodies. AMG531 (from Amgen) is one such TPO mimetic peptide consisting of a human antibody Fc carrier domain to which 2 identical peptides, each with 2 binding sites to the thrombopoietin receptor, are linked (Bussel et al. 2006). The most frequent adverse events were headache, upper respiratory tract infection, and fatigue (Kuter et al. 2006). Other proposed treatments of thrombocytopenia include using peptides such as those disclosed in WO 98/25966 and US 6,403,553 (polypeptides from the MIP class; macrophage inflammatory proteins), US 5,037,804 (a muramyldipeptide derivative). Non-peptide TPO-mimetics are disclosed in, e.g., WO 00/28987. Antibodies being tested include Anti-D. Anti-D is an antibody to the Rh (D) antigen on red blood cells. When anti-D attaches to the Rh (D) antigen, immune-mediated destruction of platelets is prevented, helping to alleviate low platelet levels in people with thrombocytopenia (Ware & Zimmerman 1998). Another possible therapy is disclosed in US 5,310,550 and relates to a composition containing human B cell differentiation factor (BCDF) in combination with IL-3. Also a parathyroid hormone (PTH) or a derivative thereof have been disclosed for use in treating thrombocytopenia (US 6,956,022), as well as IL-1 or derivatives thereof (US 5,120,534). Currently, Neumega (oprevlekin; recombinantly produced IL-11) is the only FDA approved drug for treating or preventing thrombodytopenia and it has not been very widely used.

The role of PACAP (pituitary adenylate cyclase activating peptide) in platelet activation has been disclosed previously. In particular, increased PACAP levels are correlated with thrombocytopenia and decreased platelet activation and, thus, with an increased bleeding tendency. Blocking PACAP-function by means of a polyclonal or monoclonal anti-PACAP antibodies was reported to counteract the negative effects on platelet aggregation (Freson et al. 2004; International Patent Application Publication No. WO 2004/062684). VPAC1, or VIP (vaso-intestinal peptide)/PACAP receptor 1, is G protein-coupled receptor. It is a so-called type II PACAP receptor to which both PACAP and VIP bind with similar affinity and, when activated, stimulates intracellular cAMP production. VPAC2 is another type II PACAP receptor to which both PACAP and VIP bind with similar affinity and, when activated, it stimulates cAMP turnover. PAC1 is a type I PACAP receptor to which PACAP binds with an at least 1000-fold higher affinity than VIP. When activated, it also stimulates cAMP production. PACAP and its receptors have been extensively reviewed by Vaudry et al. (2000).

VPAC1 is a 7-transmembrane receptor with, at the extracellular side the N-terminal domain and 3 extracellular loops, and at the intracellular side 3 cytoplasmic loops and the C-terminal domain. A number of VPAC1-binding antibodies are known in the art. Goetzl et al. (1994) discloses rabbit polyclonal antibody preparations raised against an N-terminal peptide covering amino acids 122 to 134 of VPAC1, against a peptide covering amino acids 191 to 222 of the first extracellular loop of VPAC1, and against a peptide covering amino acids 391 to 457 of the intracellular C-terminal domain of VPAC1. None of these antibody preparations was able to interfere with binding of VIP to VPAC1, nor did they have any effect on cellular cAMP content by their own. The antibody preparations to the extracellular loop peptide and to the C-terminal domain were reported to be able to counteract VIP-induced cAMP production, but only at low VIP-concentrations. Some of these antibody preparations have been used for immunohistochemistry, studying the effect on HIV-infectivity or in immunoaffinity applications (Busto et al. 2000, Branch et al. 2002, Shreeve 2002). Other anti-VPAC1 antibodies that have been disclosed include a rabbit polyclonal antibody preparation raised against a C-terminal VPAC1-peptide consisting of amino-acids 438-457 and used for immunohistochemistry (Schulz et al. 2004), a murine monoclonal antibody to the N-terminal VPAC1 domain (antigen not defined further) used for immunoprecipitation purposes (Langlet et al. 2005) and a rabbit polyclonal antibody to the N-terminal VPAC1 domain (antigen not defined further) used for immunohistochemical purposes (Fahrenkrug et al. 2000). Anti-VPAC1 antibodies are also commercially available and include rabbit polyclonal antibodies to the 1^{st} extracellular loop (Acris Antibodies), the 3^{rd} cytoplasmic loop and the C-terminal domain of VPAC1 (Acris Antibodies; Abcam), the N-terminal amino acids 31 to 160 of human VPAC1 (Santa Cruz Biotechnology). For other commercially available anti-VPAC1 antibodies it is unclear which antigen has been used to raise them. And for all of these antibodies information is available only for their application in western blotting, immunofluoresence, immunohistochemistry, and/or low cytometry.

Despite the progress made, the development of drugs to increase platelet production still is a standing objective of the biopharmaceutical industry.

### SUMMARY OF THE INVENTION

The current invention relates in a first aspect to a monoclonal antibody to the VPAC1 receptor wherein said antibody is specifically binding to an extracellular domain of the VPAC1 receptor, or a functionally equivalent fragment of said antibody, or a functional derivative of any thereof. More particular, the invention pertains to a monoclonal antibody to the VPAC1 receptor wherein said antibody is specifically binding to an extracellular loop, more in particular to extracellular loop 2 and/or to extracellular loop 3 of said VPAC1 receptor, or a functionally equivalent fragment of said antibody, or a functional derivative of any thereof. Alternatively, the antibody or fragment thereof, or derivative of any thereof, according to the invention is, upon binding *in vitro* to the VPAC1 receptor on VPAC1 receptor-carrying cells, decreasing cAMP levels in said cells. In a further alternative, the antibody or fragment thereof, or derivative of any thereof, according to the invention is enhancing maturation of *in vitro* cultured immature megakaryocyte cells.

In a first embodiment, the antibody or fragment thereof, or derivative of any thereof, according to the invention is binding to the VPAC1 receptor extracellular loop 2 epitope comprised in SEQ ID NO:1, binding to a protein comprising said epitope, or binding to a protein comprising SEQ ID NO:1. In a second embodiment, the antibody or fragment thereof, or derivative of any thereof, according to the invention is binding to the VPAC1 receptor extracellular loop 3 epitope comprised in SEQ ID NO:2, binding to a protein comprising said epitope, or binding to a protein comprising SEQ ID NO:2. In a further embodiment, the antibody or fragment thereof, or derivative of any thereof, according to the invention is binding both to (i) to the VPAC1 receptor extracellular loop 2 epitope comprised in SEQ ID NO:1, binding to a protein comprising said epitope, or binding to a protein comprising SEQ ID NO:1, and to (ii) the VPAC1 receptor extracellular loop 3 epitope comprised in SEQ ID NO:2, binding to a protein comprising said epitope, or binding to a protein comprising SEQ ID NO:2.

In a further aspect of the invention, the antibody or fragment thereof, or derivative of any thereof, according to the invention is characterized by comprising at least one of the CDR amino acid sequences chosen from SEQ ID NOs: 3-8 or at least one of said CDR amino acid sequences wherein 1 amino acid is changed.

In an embodiment thereto, the antibody or fragment thereof, or derivative of any thereof, according to the invention is characterized by comprising at least the heavy-chain variable region defined by SEQ ID NO:9, or by comprising a heavy-chain variable region having at least 83,5% identity to SEQ ID NO:9 outside the CDRs, and wherein the CDR amino acid sequences are defined by SEQ ID NOs: 3-5, or by said CDR amino acid sequences wherein 1 amino acid is changed, or by any combination of unchanged and changed CDRs.

In yet another embodiment the invention defines the antibody or fragment thereof, or derivative of any thereof, according to the invention as being characterized by comprising at least the light-chain variable region defined by SEQ ID NO:10, or by comprising a light-chain variable region having at least 90% identity to SEQ ID NO:10 outside the CDRs, and wherein the CDR amino acid sequences are defined by SEQ ID NOs:6-8 or by said CDR amino acid sequences wherein 1 amino acid is changed, or by any combination of unchanged and changed CDRs.

A further aspect of the invention relates to a murine monoclonal antibody which is produced by a hybridoma cell line with biological deposit accession number LMBP 6579CB; or a functionally equivalent fragment of said antibody, or a functional derivative of any thereof. Alternatively, the invention relates to a monoclonal antibody to the VPAC1 receptor which is specifically binding to said VPAC1 receptor as bound by an antibody as described above, or a functionally equivalent fragment of said monoclonal antibody, or a functional derivative of any thereof. In particular, a humanized monoclonal antibody, or a functionally equivalent fragment thereof, or a functional derivative of any thereof, is envisaged. Such humanized monoclonal antibody may be having a human IgG1-type heavy chain with the sequence defined by SEQ ID NO:11, a human IgG1-type light chain with the sequence defined by SEQ ID NO:12, a human IgG4-type heavy chain with the sequence defined by SEQ ID NO:13, or a human IgG4-type light chain with the sequence defined by SEQ ID NO:14, or may be a functionally equivalent fragment of any of said humanized antibodies, or a functional derivative of any thereof.

Yet another aspect of the invention relates to antibodies according to the invention which are binding the VPAC1 receptor defined in SEQ ID NO:15 with an affinity constant of at least 1e+8 M⁻¹, or to functionally equivalent fragments of such antibodies, or to functional derivatives of any thereof.

In particular, the functionally equivalent fragment of an antibody of the invention is a Fab, F(ab')2, scFv fragment, or nanobody, or a functional derivative thereof.

The invention further relates to compositions comprising an antibody or fragment thereof, or derivative of any thereof, according to the invention and at least one of a diluent, carrier or adjuvant. The invention also relates to an antibody or fragment thereof, or derivative of any thereof, according to the invention for use as medicament.

Another aspect of the invention covers hybridoma cell lines expressing an antibody or fragment thereof according to the invention, in particular the hybridoma cell line with biological deposit accession number LMBP 6579CB.

The invention also relates to isolated nucleic acid sequences encoding an antibody or fragment thereof according to the invention. Said nucleic acid sequences can be comprised in a recombinant vector, in particular an expression vector.

Yet another aspect of the invention relates to recombinant host cells expressing an antibody or fragment thereof according to the invention.

The invention further relates to methods of producing the antibody or fragment thereof according to the invention by recombinant expression or chemical synthesis.

In a further aspect the invention relates to the use of an antibody binding to the VPAC1 receptor or of a functionally equivalent fragment of said antibody for the preparation of a medicament:
- to stimulate maturation of immature megakaryocytes in a mammal, in particular a human;
- to treat a thrombocytopenic mammal, in particular a human; and/or
- to reduce thrombocytopenia in a mammal, in particular a human, subjected to thrombocytopenia-inducing conditions.

In particular, in said uses, said medicament can be administered prior to, concomitant with or after administration of a further compound. In other words, said medicament can be part of of a treatment wherein several medicines are combined. Said further compounds include thrombopoietin, AMG 531, or Interleukin-11, or any compound known to have a positive effect on megakaryocyte maturation.

In one embodiment, any of the above medicaments may be prepared with an antibody or a functionally equivalent fragment thereof that is binding to an extracellular domain of the VPAC1 receptor. In particular this antibody or fragment thereof can be any antibody or fragment thereof as extensively described supra. In particular it can be a monoclonal antibody or a functionally equivalent fragment thereof.

### FIGURE LEGENDS

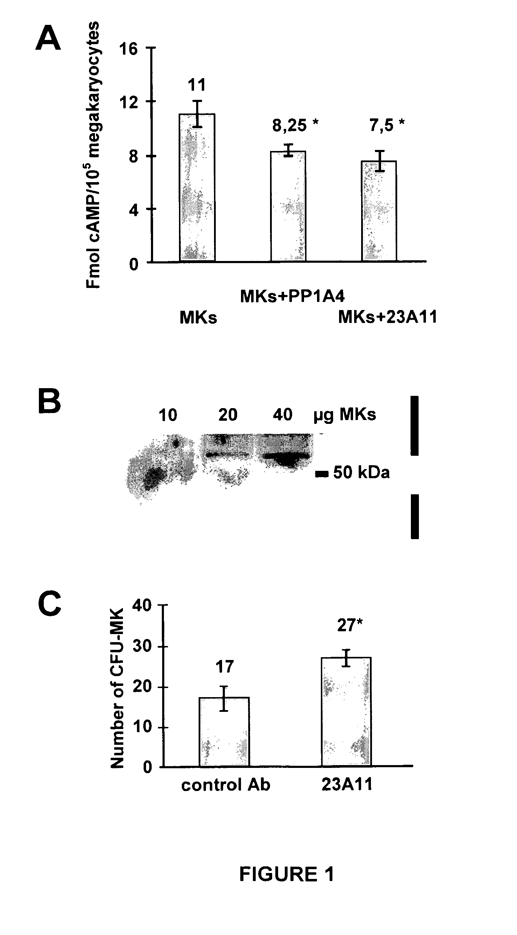

**FIGURE 1****. Effect of anti-VPAC1 monoclonal antibody 23A11 on the in vitro megakaryopoiesis.**
   **Figure 1A****.** Basal cAMP levels in mice bone marrow-derived CD41/61⁺ megakaryocytes in the absence or presence of anti-PACAP monoclonal antibody PP1A4 or anti-VPAC1 monoclonal antibody 23A11. Bars represent the mean ± SD (**P* < 0.05).
   **Figure 1** **B.** Immunoblot analysis of VPAC1 expression in mouse bone marrow-derived CD41/61⁺ megakaryocytes.
   **Figure 1C****.** Sca1⁺ murine bone marrow cells incubated with 23A11 resulted in increased numbers of CFU-MKs after 12 days. Bars represent the mean ± SD (P < 0.01).
**FIGURE 2****. Effect of anti-VPAC1 monoclonal antibody 23A11 on the in** vitro megakaryopoiesis.
   FACS analysis showing the percentage of CD41⁺ megakaryocytes derived from human CD34⁺ cord blood cells in the absence (left two panels) or presence (right two panels) of anti-VPAC1 monoclonal antibody 23A11, on day 5 (upper two panels) or day 14 (lower two panels).
**FIGURE 3****. Effect of anti-VPAC1 monoclonal antibody on busulfan-induced thrombocytopenia.**
   **Figure 3A****.** Platelet count in mice following administration of anti-VPAC1 monoclonal antibody 23A11 (filled squares) or PBS (filled circles) on days 0, 3 and 7 and busulfan treatment on days 7 and 10. Each point represents the mean platelet count from 5 (filled squares) or 3 (filled circles) mice ± SD. **P < 0.01 by ANOVA.
   **Figure 3B****.** D. Platelet count in rabbits (n=3 in each group) following administration of anti-VPAC1 monoclonal antibody 23A11 (filled squares) or PBS (filled circles) on days 0, 3 and 7 and 10 and busulfan treatment on days 7 and 10. Each point represents the mean platelet count ± SD. **P* <0.05; *^{#}P* < 0.001; by ANOVA.
**FIGURE 4****. Effect of anti-VPAC1 monoclonal antibody on human thrombocytopenia due to GATA1 defect.**
   Histograms demonstrate the DNA ploidy distribution of CD41⁺ megakaryocytes differentiated from CD34⁺ cells from bone marrow of the GATA1-D218Y patient in the absence (A) or presence of 23A11 (B) on day 12. FACS results are representative of 2 separate analyses.
**FIGURE 5****. Amino acid sequences of murine monoclonal anti-VPAC1 antibody 23A11.**
   Depicted are the amino acid sequences of the heavy chain (top; SEQ ID NO:9) and the light chain (bottom; SEQ ID NO:10) variable regions. Within the heavy-and light chain variable regions the CDR sequences are underlined and reference is made to the corresponding SEQ ID NOs.

### DETAILED DESCRIPTION OF THE INVENTION

As discussed above, there is still a need for candidate drugs that have a good potential to prevent, treat or reduce thrombocytopenia. The current invention provides such candidate drugs in the form of anti-VPAC1 antibodies. Indeed, although many anti-VPAC1 antibodies, mostly polyclonal, are available in the art, none of them has actually been shown to be able to prevent, treat or reduce thrombocytopenia.

"Thrombocytopenia" or a low platelet count can be the consequence of different reasons. It can be the result of a reduced production of platelets because of a problem involving the bone marrow. As such, thrombocytopenia may be associated with leukemia, some types of anemia, viral infections (including HCV or HIV infection), cancers that affect bone marrow, chemotherapy drugs and heavy alcohol consumption. Thrombocytopenia may also be caused by an increased breakdown of platelets such as in the idiopathic thrombocytopenic purpura (ITP) condition in which the immune system mistakenly forms antiplatelet antibodies. Besides ITP, other autoimmune diseases, such as lupus and rheumatoid arthritis, may lead to destruction of platelets due to a malfunctioning immune system. Other conditions that may cause thrombocytopenia include blood poisoning (septicemia; from severe bacterial infections), thrombotic thrombocytopenic purpura (TTP), a rare, life-threatening condition that occurs when small blood clots suddenly form throughout the body (using up large numbers of platelets) and hemolytic uremic syndrome, another rare disorder that causes a sharp drop in platelets, destruction of red blood cells and impairment of kidney function. Finally also pregnancy may cause mild thrombocytopenia. Certain medications can cause a thrombocytopenic reaction by confusing the immune system and causing it to destroy platelets. Examples include heparin, quinidine, quinine, sulfa-containing antibiotics, some oral diabetes drugs, gold salts and rifampin. Sometimes, heparin-induced thrombocytopenia can cause excessive blood clotting instead of bleeding, increasing the risk of clot formation deep within a leg blood vessel or the transport of such a clot to the lungs, which can be life-threatening. Other medications, such as used in chemotherapy, or radiation may also cause a drop in the number of platelets. Thrombocytopenia may also be associated with genetic defects such as GATA1 deficiency.

Based on Examples 2 to 4 herein, the current invention relates in a first aspect to a monoclonal antibody to the VPAC1 receptor wherein said antibody is specifically binding to an extracellular domain of the VPAC1 receptor, or a functionally equivalent fragment of said antibody, or a functional derivative of any thereof. In particular the invention pertains to a monoclonal antibody binding to an extracellular loop, more in particular to extracellular loop 2 and/or to extracellular loop 3 of said VPAC1 receptor, or a functionally equivalent fragment of said antibody, or a functional derivative of any thereof. Alternatively, the antibody or fragment thereof, or derivative of any thereof, according to the invention is, upon binding *in vitro* to the VPAC1 receptor on VPAC1 receptor-carrying cells, decreasing cAMP levels in said cells. VPAC1 receptor-carrying cells include, but are not limited to megakaryocytes. In a further alternative, the antibody or fragment thereof, or derivative of any thereof, according to the invention is enhancing maturation of *in vitro* cultured immature megakaryocyte cells. In this context it is noted that said antibodies to the VPAC1 receptor surprisingly do not seem to prevent binding of PACAP to the VPAC1 receptor. Two further surprising elements reside in the facts that antibodies to the VPAC1 receptor are more potent in lowering cAMP levels, and have a greater beneficial effect on megakaryocyte stimulation than anti-PACAP antibodies. Antibodies to the VPAC1 receptor, as herein described, thus intrinsically are more efficient than anti-PACAP antibodies for reducing, treating or preventing thrombocytopenia.

The term "antibody" refers to a protein or polypeptide having specificity and affinity for an antigen or for an antigenic determinant. Such an antibody is commonly composed of 4 chains, 2 heavy- and 2 light chains, and is thus tetrameric. An exception thereto are camel antibodies that are composed of heavy chain dimers and are devoid of light chains, but nevertheless have an extensive antigen-binding repertoire; such antibodies are also termed nanobodies. An antibody usually has both variable and constant regions whereby the variable regions are mostly responsible for determining the specificity of the antibody and will comprise complementarity determining regions (CDRs). Non-human mammalian antibodies or animal antibodies can be humanized (see for instance Winter and Harris 1993). The antibodies or monoclonal antibodies according to the invention may be humanized versions of for instance rodent antibodies or rodent monoclonal antibodies. Humanisation of antibodies entails recombinant DNA technology, and is departing from parts of rodent and/or human genomic DNA sequences coding for H and L chains or from cDNA clones coding for H and L chains. Techniques for humanization of non-human antibodies are known to the skilled person as these form part of the current state of the art.

The term "antigen" refers to a structure, often a polypeptide or protein, for which an immunoglobulin, such as an antibody, has affinity and specificity.

The terms "antigenic determinant", "antigenic target" and "epitope" all refer to a specific binding site on an antigen or on an antigenic structure for which an immunoglobulin, such as an antibody, has specificity and affinity. An epitope usually consists of at least 3, 4, 5, 6, 7, 8, 9, or 10 amino acids. An epitope can be linear (or contiguous) or conformational (or discontiguous).

The term "specificity" refers to the ability of an immunoglobulin, such as an antibody, to bind preferentially to one antigenic target versus a different antigenic target and does not necessarily imply high affinity.

The term "affinity" refers to the degree to which an immunoglobulin, such as an antibody, binds to an antigen so as to shift the equilibrium of antigen and antibody toward the presence of a complex formed by their binding. Thus, where an antigen and antibody are combined in relatively equal concentration, an antibody of high affinity will bind to the available antigen so as to shift the equilibrium toward high concentration of the resulting complex.

The term "complementarity determining region" or "CDR" refers to variable regions of either H (heavy) or L (light) chains (also abbreviated as VH and VL, respectively) and contains the amino acid sequences capable of specifically binding to antigenic targets. These CDR regions account for the basic specificity of the antibody for a particular antigenic determinant structure. Such regions are also referred to as "hypervariable regions." The CDRs represent non-contiguous stretches of amino acids within the variable regions but, regardless of species, the positional locations of these critical amino acid sequences within the variable heavy and light chain regions have been found to have similar locations within the amino acid sequences of the variable chains. The variable heavy and light chains of all canonical antibodies each have 3 CDR regions, each non- contiguous with the others (termed L1, L2, L3, H1, H2, H3) for the respective light (L) and heavy (H) chains. The accepted CDR regions have been described by Kabat et al. (1991).

The term "functionally equivalent fragment of an antibody" refers to a portion of an antibody that by itself has specificity and affinity for an antigenic determinant, or epitope, and contains one or more CDRs accounting for such specificity. Non-limiting examples include Fab, Fab', F(ab)'2, scFv, heavy-light chain dimers, nanobodies, domain antibodies, and single chain structures, such as a complete light chain or complete heavy chain. An additional requirement for "functional equivalence" of said fragments in the light of the present invention may be, alternatively, that said fragments are capable of decreasing cAMP levels upon binding *in vitro* to VPAC1 in VPAC1-carrying cells, or are capable of enhancing maturation of *in vitro* cultured immature megakaryocyte cells.

Derivatives of the antibodies of the invention, or of functional equivalent fragments of said antibodies, include, but are not limited to antibodies or fragments thereof labeled with an appropriate label, said label can for instance be of the enzymatic, colorimetric, chemiluminescent, fluorescent, or radioactive type. A "functional derivative" of an antibody of the invention, or of a functionally equivalent fragment of said antibody, is a derivative complying with the definition of functional equivalence as described supra.

As outlined in Example 9, the anti-VPAC1 receptor antibodies of the invention are binding to isolated extracellular loop 2 of the VPAC1 receptor (18 amino acids; see SEQ ID NO:1) as well as to isolated extracellular loop 3 of the VPAC1 receptor (13 amino acids; see SEQ ID NO:2). Further delineation of the epitope(s) in these extracellular loops is a matter of limited and routine experimentation and could be performed, e.g., by chemical synthesis of a limited set of overlapping peptides (e.g., 5-mers, 6-, 7-, 8- or 9-mers) and screening those for binding to the anti-VPAC1 receptor antibodies of the invention. In a similar way, larger proteins containing the defined epitope(s) or SEQ ID NOs: 1 and/or 2 can be made synthetically or via recombinant expression and subsequently screened for binding to the anti-VPAC1 receptor antibodies of the invention. As a skilled artisan will understand, this is all a matter of routine experimentation.

Therefore, a first embodiment of the invention defines the antibody or fragment thereof, or derivative of any thereof, according to the invention further as binding to the VPAC1 receptor extracellular loop 2 epitope comprised in SEQ ID NO:1, binding to a protein comprising said epitope, or binding to a protein comprising SEQ ID NO:1.

In a second embodiment, the antibody or fragment thereof, or derivative of any thereof, according to the invention is binding to the VPAC1 receptor extracellular loop 3 epitope comprised in SEQ ID NO:2, binding to a protein comprising said epitope, or binding to a protein comprising SEQ ID NO:2.

In a further embodiment, the antibody or fragment thereof, or derivative of any thereof, according to the invention is binding both to (i) to the VPAC1 receptor extracellular loop 2 epitope comprised in SEQ ID NO:1, binding to a protein comprising said epitope, or binding to a protein comprising SEQ ID NO:1, and to (ii) the VPAC1 receptor extracellular loop 3 epitope comprised in SEQ ID NO:2, binding to a protein comprising said epitope, or binding to a protein comprising SEQ ID NO:2.

Example 7 herein describes the molecular characterization of the anti-VPAC1 receptor antibodies according to the invention, as well as the humanization to human-type IgG1 and IgG4 antibodies. As the humanized antibodies, or fragments thereof, are still binding to the VPAC1 receptor, the humanization process indicated that the variable regions can accommodate amino acid variation without loosing binding of the resulting antibody, or fragments thereof, to their target epitope. Over the whole variable regions, not taking the CDRs into account for calculating % identity, the heavy chain is at least 80%, 81%, 82%, 83%, 83,5%, 84%, 85%, 87,5%, 88%, 89%, 90%, 91%, 92%, 93 %, 94%, 95%, 96%, 97%, 98%, 99% or 100% identical to the murine heavy chain of SEQ ID NO:9; the light chain can be at least 80%, 85%, 87,5%, 90%, 91%, 92%, 93 %, 94%, 95%, 96%, 97%, 98%, 99% or 100% identical to the murine light chain of SEQ ID NO:10. In particular the following amino acid substitutions can be introduced:
- in murine heavy chain variable region (SEQ ID NO:9): Ser at position 10 to Gly; Ser at position 23 to Thr; Thr at position 25 to Ser; Phe at position 40 to His; Asn at position 43 to Lys; Lys at position 44 to Gly; Ala at position 62 to Ser; Ser at position 68 to Thr; Thr at position 70 to Ser; Tyr at position 79 to Ser; Gln at position 81 to Lys; Asn at position 83 to Ser; Thr at position 87 to Ala; and Glu at position 88 to Ala;
- in murine light chain variable region (SEQ ID NO:10): Asp at position 7 to Ser; Glu at position 8 to Pro; Asn at position 11 to Leu; Ser at position 15 to Pro; Ser at position 18 to Pro; Glu at position 79 to Lys; Lys at position 84 to Glu; Ala at position 105 to Gln, and Leu at position 111 to Ile;
- wherein the positions of the amino acids are relative to SEQ ID NOs:9 and 10, respectively.

Furthermore, limited changes in the CDR regions themselves are expected not to change drastically binding of the resulting antibodies, or fragments thereof, to the VPAC 1 receptor.

Therefore, in a further aspect of the invention, the antibody or fragment thereof, or derivative of any thereof, according to the invention is characterized by comprising at least one of the CDR amino acid sequences chosen from SEQ ID NOs: 3-8 or at least one of said CDR amino acid sequences wherein 1 amino acid is changed. Herein SEQ ID NO:3 corresponds to CDR H1, SEQ ID NO:4 to CDR H2, SEQ ID NO:5 to CDR H3, SEQ ID NO:6 to CDR L1, SEQ ID NO:7 to CDR L2, and SEQ ID NO:8 to CDR L3. "At least one of the CDR amino acid sequences" is to be read as 1, 2, 3, 4, 5 or all 6 of said CDR amino acid sequences wherein, if more than 1, the order of the CDR amino acid sequences does not need to be the order as occurring in the original antibody. Likewise, if comprising more than one of said CDRs, any combination of unchanged (as in SEQ ID NOs:3-8) or changed (1 amino acid change in any of SEQ ID NOs:3-8) CDR amino acid sequences is envisaged. In a further embodiment the amino acid changes in the CDR sequences, or, by extension, in the variable regions, may, but must not be limited to conservative amino acid changes or substitutions (within the amino acid groups P,A,G,S,T,C; V,I,L,M; W,F,Y; N,Q,D,E; and K,H,R).

Thus, in one embodiment to the latter aspect of the invention, the antibody or fragment thereof, or derivative of any thereof, according to the invention is characterized by comprising at least the heavy-chain variable region defined by SEQ ID NO:9, or by comprising a heavy-chain variable region having at least 83,5% identity to SEQ ID NO:9 outside the CDRs, and wherein the CDR amino acid sequences are defined by SEQ ID NOs: 3-5 or by said CDR amino acid sequences wherein 1 amino acid is changed, or by any combination of unchanged and changed CDRs. The above-indicated % identity relates to the heavy-chain variable region without the CDRs (or outside the CDRs).

In yet another embodiment the invention defines the antibody or fragment thereof, or derivative of any thereof, according to the invention as being characterized by comprising at least the light-chain variable region defined by SEQ ID NO:10, or by comprising a light-chain variable region having at least 90% identity to SEQ ID NO:10 outside the CDRs, and wherein the CDR amino acid sequences are defined by SEQ ID NOs:6-8 or by said CDR amino acid sequences wherein 1 amino acid is changed, or by any combination of unchanged and changed CDRs. The above-indicated % identity relates to the light-chain variable region without the CDRs (or outside the CDRs).

A further aspect of the invention relates to a murine monoclonal antibody which is produced by a hybridoma cell line with biological deposit accession number LMBP 6579CB, or a functionally equivalent fragment of said antibody, or a functional derivative of any thereof.

Alternatively, the invention relates to a monoclonal antibody to the VPAC1 receptor which is specifically binding to said VPAC1 receptor as bound by any of the antibodies as described above, or a functionally equivalent fragment of said monoclonal antibody, or a functional derivative of said monoclonal antibody or said fragment thereof. In particular said monoclonal antibody is specifically binding to extracellular loop 2 and/or to extracellular loop 3 of the VPAC1 receptor.

In particular, a humanized monoclonal antibody, or a functionally equivalent fragment thereof, or a functional derivative of any thereof, is envisaged. Such humanized monoclonal antibody may be having a human IgG1-type heavy chain with the sequence defined by SEQ ID NO:11, a human IgG1-type light chain with the sequence defined by SEQ ID NO:12, a human IgG4-type heavy chain with the sequence defined by SEQ ID NO:13, or a human IgG4-type light chain with the sequence defined by SEQ ID NO:14, or may be a functionally equivalent fragment of any of said humanized antibodies, or a functional derivative of any thereof.

Yet another aspect of the invention, based on Example 8 herein, relates to antibodies according to the invention which are binding the VPAC1 receptor defined in SEQ ID NO:15 with an affinity constant between 1e+8 M⁻¹ and 10e+7 M⁻¹, between 1e+8 M⁻¹ and 5e+7 M⁻¹, or with an affinity constant of at least 1e+8 M⁻¹, at least 2,5e+7 M⁻¹ or at least 5e+7 M⁻¹. Functionally equivalent fragments of such antibodies, or functional derivatives of said antibodies or fragments are also envisaged.

The invention further relates to compositions comprising an antibody or functionally equivalent fragment thereof, or functional derivative of any thereof, according to the invention and at least one of a diluent, carrier or adjuvant. In particular, said composition is comprising an effective amount of said antibody, fragment or derivative.

A "diluent, carrier or adjuvant", is any suitable excipient, diluent, carrier and/or adjuvant which, by themselves, do not induce the production of antibodies harmful to the individual receiving the composition.

A (pharmaceutically acceptable) carrier or adjuvant may enhance the response elicited by an antibody or fragment thereof according to the invention, e.g., by providing a continuous release of the antibody or fragment thereof according to the invention over a prolonged period of time (slow-release formulations). Suitable carriers or adjuvantia typically comprise one or more of the compounds included in the following non-exhaustive list:
- large slowly metabolized macromolecules such as proteins, polysaccharides, polylactic acids, polyglycolic acids, polymeric amino acids, amino acid copolymers and inactive virus particles;
- aluminium hydroxide, aluminium phosphate (see International Patent Application Publication No. WO93/24148), alum (KAI(SO₄)₂.12H₂O), or one of these in combination with 3-0-deacylated monophosphoryl lipid A (see International Patent Application Publication No. WO93/19780);
- N-acetyl-muramyl-L-threonyl-D-isoglutamine (see U.S. Patent No. 4,606,918), N-acetyl-normuramyl-L-alanyl-D-isoglutamine, N-acetylmuramyl-L-alanyl-D-isoglutamyl-L-alanine2-(1',2'-dipalmitoyl-sn-glycero-3-hydroxyphosphoryloxy) ethylamine;
- RIBI (ImmunoChem Research Inc., Hamilton, MT, USA) which contains monophosphoryl lipid A (i.e., a detoxified endotoxin), trehalose-6,6-dimycolate, and cell wall skeleton (MPL + TDM + CWS) in a 2% squalene/Tween 80 emulsion. Any of the three components MPL, TDM or CWS may also be used alone or combined 2 by 2;
- adjuvants such as Stimulon (Cambridge Bioscience, Worcester, MA, USA), SAF-1 (Syntex);
- adjuvants such as combinations between QS21 and 3-de-O-acetylated monophosphoryl lipid A (see International Patent Application Publication No. WO94/00153) which may be further supplemented with an oil-in-water emulsion (see, e.g., International Patent Application Publication Nos. WO95/17210, WO97/01640 and WO9856414) in which the oil-in-water emulsion comprises a metabolisable oil and a saponin, or a metabolisable oil, a saponin, and
- a sterol, or which may be further supplemented with a cytokine (see International Patent Application Publication No. WO98/57659);
- adjuvants such as MF-59 (Chiron), or poly[di(carboxylatophenoxy) phosphazene] based adjuvants (Virus Research Institute);
- blockcopolymer based adjuvants such as Optivax (Vaxcel, Cytrx) or inulin-based adjuvants, such as Algammulin and Gammalnulin (Anutech);
- Complete or Incomplete Freund's Adjuvant (CFA or IFA, respectively) or Gerbu preparations (Gerbu Biotechnik). It is to be understood that Complete Freund's Adjuvant (CFA) may be used for non-human applications and research purposes as well;
- a saponin such as QuilA, a purified saponin such as QS21, QS7 or QS17, β-escin or digitonin;
- immunostimulatory oligonucleotides comprising unmethylated CpG dinucleotides such as [purine-purine-CG-pyrimidine-pyrimidine] oligonucleotides. These immunostimulatory oligonucleotides include CpG class A, B, and C molecules (Coley Pharmaceuticals), ISS (Dynavax), lmmunomers (Hybridon). Immunostimulatory oligonucleotides may also be combined with cationic peptides as described, e.g., by Riedl et al. (2002);
- Immune Stimulating Complexes comprising saponins, for example Quil A (ISCOMS);
- a biodegradable and/or biocompatible oil such as squalane, squalene, eicosane, tetratetracontane, glycerol, peanut oil, vegetable oil, in a concentration of, e.g., 1 to 10% or 2.5 to 5%;
- vitamins such as vitamin C (ascorbic acid or its salts or esters), vitamin E (tocopherol), or vitamin A;
- carotenoids, or natural or synthetic flavanoids;
- trace elements, such as selenium;
- a Toll-like receptor ligand as reviewed in Barton and Medzhitov (2002).

Any of the afore-mentioned adjuvants comprising 3-de-O-acetylated monophosphoryl lipid A, said 3-de-O-acetylated monophosphoryl lipid A may be forming a small particle (see International Patent Application Publication No. WO94/21292).

In any of the aforementioned adjuvants MPL or 3-de-O-acetylated monophosphoryl lipid A can be replaced by a synthetic analogue referred to as RC-529 or by any other amino-alkyl glucosaminide 4-phosphate (Johnson et al. 1999, Persing et al. 2002). Alternatively it can be replaced by other lipid A analogues such as OM-197 (Byl et al. 2003).

A "diluent", or more in particular a "pharmaceutically acceptable diluent", includes diluents such as water, saline, physiological salt solutions, glycerol, ethanol, etc. Auxiliary substances such as wetting or emulsifying agents, pH buffering substances, preservatives may be included in such diluents.

The composition according to the invention may be prepared as an injectable, either as a liquid solution or suspension. Injection may be subcutaneous, intramuscular, intravenous, intra-arterial, intraperitoneal, intrathecal, intradermal, intraepidermal. The composition may also be prepared to make it suitable for other types of administration such as implantation, suppositories, oral ingestion, enteric application, inhalation, aerosolization, nasal spray or drops, or administration through medical devices such as stents. Solid forms, suitable for dissolving in, or suspension in, liquid vehicles prior to injection may also be prepared. The preparation may also be emulsified or encapsulated in liposomes for enhancing its effect. The preparation may be administered to a subject as a bolus dose or by continuous infusion. The preparation may also be administered continuously via an osmotic minipump.

An "effective amount" of an active substance in a composition is the amount of said substance required and sufficient to elicit an adequate response in preventing or treating or reducing the targeted medical indication. It will be clear to the skilled artisan that such response may require successive (in time) administrations with the composition as part of a administration scheme or - schedule. The "effective amount" may vary depending on the health and physical condition of the individual to be treated, the age of the individual to be treated (e.g. dosing for infants may be lower than for adults) the taxonomic group of the individual to be treated (e.g. human, non-human primate, primate, etc.), the capacity of the individual's system to respond effectively, the degree of the desired response, the formulation of the active substance, the treating doctor's assessment and other relevant factors. It is expected that the effective amount of the active substance of the invention (anti-VPAC1 antibody or functionally equivalent fragment thereof, or functional derivative of any thereof) will fall in a relatively broad range that can be determined through routine trials. Usually, the amount will vary from 0.01 to 1000 µg/dose, more particularly from 0.1 to 100 µg/dose. Alternatively, the active substance may be administered at a dose between 1 µg/kg body weight and 10 mg/kg, or between 10 µg/kg and 5 mg/kg, or between 100 µg/kg and 2 mg/kg. Dosage treatment may be a single dose schedule or a multiple dose schedule. If the active substance is administered continuously, administered doses may be between 1 and 100 µg/kg/minute, between 1 and 50 µg/kg/minute, between 5 and 50 µg/kg/minute, or between 5 and 20 µg/kg/minute.

The invention also relates to an antibody or fragment thereof, or derivative of any thereof, according to the invention for use as medicament. The applicability of the antibody or fragment thereof, or derivative of any thereof, as medicament is supported by Examples 4 to 6 herein.

Another aspect of the invention covers hybridoma cell lines expressing an antibody or fragment thereof according to the invention, in particular the hybridoma cell line with biological deposit accession number LMBP 6579CB.

The invention also relates to isolated nucleic acid sequences encoding an antibody or fragment thereof according to the invention. Said nucleic acid sequences can be comprised in a recombinant vector, in particular an expression vector.

Yet another aspect of the invention relates to recombinant host cells expressing an antibody or fragment thereof according to the invention. Said host cell can be any cell capable of expressing said antibody or fragment thereof. Suitable host cells include, but are not limited to, cultured mammalian (such as HEK293) or insect cells, cultured plant cells or transgenic plants, yeasts such a *Saccharomyces, Schizosaccharomyces, Pichia, Hansenula, Torulopsis,* and bacterial cells. Expression of the antibody of the invention or functionally equivalent fragment thereof may be transient or constitutive.

Methods of producing the above-described VPAC1-binding antibodies, or functionally equivalent fragments thereof, form an integral aspect of the invention. In particular, such methods can comprise the steps of:
(i) obtaining a crude preparation of said antibody or antibody fragment by means of recombinant expression of said antibody or antibody fragment, or by means of chemical synthesis of said antibody or antibody fragment;
(ii) purifying said antibody or antibody fragment from the crude preparation obtained in (i).

Alternatively, an active fragment of the antibodies of the invention binding to VPAC1 can be obtained or produced by a method comprising the steps of:
(i) obtaining a crude preparation of an antibody comprising said fragment by means of recombinant expression of said antibody or by means of chemical synthesis of the antibody ;
(ii) purifying said antibody from the crude preparation obtained in (i).
(iii) isolating the active fragment from the antibody purified in (ii).

In a further aspect the invention relates to the use of an antibody binding to the VPAC1 receptor or of a functionally equivalent fragment of said antibody for the preparation of a medicament
- to stimulate maturation of immature megakaryocytes in a mammal, in particular a human;
- to treat a thrombocytopenic mammal, in particular a human; and/or
- to reduce thrombocytopenia in a mammal, in particular a human, subjected to thrombocytopenia-inducing conditions.

Examples of thrombocytopenia-inducing conditions include irradiation and/or chemotherapy as applied in cancer therapy.

As used herein, "thrombocytopenia" is any disorder in which the platelet level in an affected individual falls below a normal range of platelet numbers for that individual, due to disturbance in production distribution or destruction. In humans, normal blood platelet levels range from about 150.000 to 300.000 per microliter peripheral blood. With a platelet level of 100.000 per microliter patients have no abnormal bleeding even with major surgery; with a platelet count of 50.000 to 100. 000 per microliter, patients may bleed longer than normal with severe trauma; with a platelet count of 20.000 to 50.000 per microliter, bleeding occurs with minor trauma but spontaneous bleeding is unusual; with a platelet count of less than 20.000, patients may have spontaneous bleeding and when the platelet count is less than 10.000 per microliter, patients are at high risk for severe bleeding. Thrombocytopenia also refers to a decrease in platelet number in an individual when compared to the platelet number measured at a certain reference point in that individual. The decrease in platelet number in the individual can be a decrease in more than 20%, 30%, 40%, 60%, 80%, 90%, 95% or even more, compared to value at the reference point. A decrease in platelet number when compared to the platelet number measured at a certain reference point, can in certain individuals be accompanied with changes in bleeding, while in other individuals a comparable decrease will not be accompanied with changes in bleeding. The reference point mentioned, can be for instance the start of a therapy such as a radiation or chemotherapy. "Reducing thrombocytopenia" refers to any improvement of platelet number from an abnormal value to a for the affected individual normal value. In particular, the term refers to improving or increasing platelet numbers to a level at which the affected individual is no longer at high risk to develop severe spontaneous bleeding.

In particular, in said uses described supra, said medicament can be administered prior to, concomitant with or after administration of a further compound. In other words, said medicament can be part of a treatment wherein several medicines are combined. Said further compounds include thrombopoietin, AMG 531, or Interleukin-11, or any compound known to have a positive effect on megakaryocyte maturation.

In one embodiment, any of the above medicaments may be prepared with an antibody or a functionally equivalent fragment thereof that is binding to an extracellular domain of the VPAC1 receptor. Said extracellular domain may in particular be an extracellular loop, or more in particular be composed of extracellular loops 2 and/or 3 of the VPAC1 receptor. In particular the antibody or fragment thereof can be any antibody or fragment thereof as extensively described supra. In particular it can be a monoclonal antibody or a functionally equivalent fragment thereof.

The invention further relates to an isolated complementarity determining region (CDR) of an anti-VPAC1 receptor antibody. In one embodiment thereto, said CDR has an amino acid sequences chosen from SEQ ID NOs: 3 to 8 or a CDR with an amino acid sequence that comprises one amino acid substitution relative to any of SEQ ID NOs: 3 to 8. Said isolated CDR can also be incorporated in a composition further comprising for instance a carrier, adjuvant, or diluent. Nucleic acid sequences encoding said CDRs are also envisaged by the invention. The isolated CDR nucleic acid sequences are part of the invention, as well as any vector or recombinant nucleic acid (DNA, RNA, PNA, LNA, or any hybrid thereof; linear or circular; independent of strandedness) comprising such CDR nucleic acid. Any host cell comprising such CDR nucleic acid sequence, vector or recombinant nucleic acid is likewise part of the invention.

The invention also relates to an isolated variable region of an anti-VPAC1 receptor antibody. In one embodiment thereto, said variable region has an amino acid sequence which is chosen from SEQ ID NOs: 9 to 10 or an amino acid sequence that is at least 81, 82, 83, 83,5%, 84, 85, 86, 87, 88, 89, 90, 91, 92, 93, 94, 95, 96, 97, 98, or 99 % identical with any of SEQ ID NOs: 9 or 10 wherein said % identity is calculated with omission of the CDR sequences (cfr. supra). Said variable region can also be incorporated in a composition further comprising for instance a carrier, adjuvant, or diluent. The isolated variable region nucleic acid sequences are also part of the invention, as well as any vector or recombinant nucleic acid (DNA, RNA, PNA, LNA, or any hybrid thereof; linear or circular; independent of strandedness) comprising such variable region nucleic acid. Any host cell comprising such variable region nucleic acid sequence, vector or recombinant nucleic acid is likewise part of the invention.

A further aspect of the invention relates to compounds capable of binding the VPAC1 receptor with said compounds comprising at least one CDR as described above or at least one variable region as described above. Such a compound can be used in stimulating megakaryocyte maturation and/or reducing or treating thrombocytopenia as described supra.

Any host cell comprising and/or secreting (i) an anti-VPAC1 receptor antibody of the invention, (ii) an active fragment of (i), (iii) a CDR amino acid sequence comprised in the antibody of (i), (iv) a variable region amino acid sequence comprised in the antibody of (i), or (v) a compound comprising (i), (ii), (iii) or (iv) is likewise part of the invention.

### EXAMPLES

### EXAMPLE 1. Antibodies.

The following antibodies were used for flow cytometry: PE-conjugated anti-CD34

(8G12), FITC-conjugated anti-CD41a (HIP8), PerCP-conjugated anti-CD61 (RUU-PL7F12), and FITC-conjugated anti-CD41/61 (Leo-D2) (Emfret Analysis, Wurzburg, Germany). The anti-PACAP antibody PP1A4 was previously described (see International Patent Application Publication WO 2004/062684).

### EXAMPLE 2. Generation of anti-VPAC1 antibodies.

The sequence coding for human VPAC1 (SEQ ID NO:15; GenBank accession number NP_004615) was cloned into the vector pGEX vector which contains the sequence coding for GST (glutathione-S-transferase) at 5'of insert. These recombinant fusion proteins were expressed in *Escherichia coli* and purified by affinity chromatography on immobilized glutathione (Amersham Biosciences, Freiburg, Germany). The primary antibodies were purified on protein A SepharoseTM beads (Amersham Biosciences) and controlled for their reactivity towards recombinant VPAC1 by ELISA. The purified fusion protein GST-VPAC1 was used for immunization of the BALB/c mice. Spleen cells of immunized mice were fused to a mouse myeloma cell line Sp2/0-Ag14 and the resulting hybridomas were screened for monoclonal antibodies binding to the VPAC1 receptor. In a second step, VPAC1-binding monoclonal antibodies were screened further for their capacity to lower cAMP levels in *in vitro* cultured cells (megakaryocytes) carrying the VPAC1 receptor.

### EXAMPLE 3. Identification of monoclonal antibody inhibiting VPAC1 receptor-mediated cAMP production.

Megakaryocytes were isolated from bone marrow pooled from 5 (polyploidy by FACS analysis) or 10 (cAMP measurements) mice by magnetic cell sorting using the FITC-conjugated CD41/61 antibody and anti-FITC magnetic beads (Miltenyi Biotec, Utrecht, The Netherlands). Basal cAMP levels in megakaryocytes were measured in the presence of the phosphodiesterase inhibitor 3-isobutyl Imethylxanthine (IBMX, 100 µM f.c.) using a cAMP enzyme-immunoassay (GE Healthcare Life Sciences, Uppsala, Sweden) as previously described (Freson et al. 2003, Freson et al. 2004). The incubation time with a monoclonal anti-VPAC1 antibody (at 10µg/ml) was 2 hours in IMDM (Iscove's modified Dulbecco medium). The monoclonal antibody 23A11 was identified as a potent inhibitor of VPAC1 receptor-mediated cAMP production. Surprisingly, as indicated in Figure 1A, the inhibitory properties of the VPAC1 receptor antibody were more potent than those of a previously described monoclonal PACAP antibody (PP1A4).

### EXAMPLE 4. Stimulation of in vitro megakaryopoiesis by VPAC1 receptor antibody.

Human CD34+ cells were isolated from bone marrow or cord blood by magnetic cell sorting (Miltenyi Biotec). We purified a Sca1+ population from bone marrow cells pooled from 5 mice using the EasySep Sca1 positive selection kit (Stem Cell Technologies, Vancouver, BC, Canada). Human CD34+ cells (5 x 10³) and mice Sca1+ cells (5 x 10⁴) were cultured in Megacult-C 04973 with cytokines (for human) and Megacult-C 04964 supplemented with recombinant cytokines according to the manufacturer's instructions (for mice) (Stem Cell Technologies). The number of megakaryocytic colonies (CFU-MK) was determined in a semi-solid culture system using a commercially available kit (StemCell Technologies). The total number of colonies was counted 12 days later using a light microscope (Leica DM RBE) in cultures performed in duplicate. Megakaryocytes were identified as large cells with lobulated nuclei and basophilic staining.

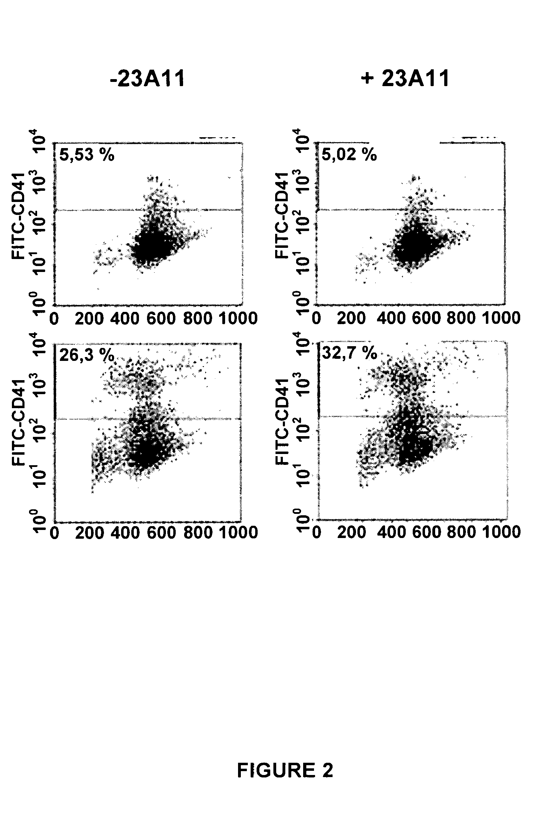

Megakaryocytes isolated from mouse bone marrow, subsequently incubated with PP1A4 or 23A11 showed decreased basal cAMP levels (Figure 1A and previous Example) and immunoblot analysis using 23A11 showed the presence of the VPAC1 receptor on the murine megakaryocytes (Figure 1 B). We therefore studied whether inhibition of VPAC1 affected megakaryopoiesis by performing in vitro CFU-MK assays. More megakaryocytes were generated from Sca1+ cells derived from normal mouse bone marrow after 12 days incubation with 23A11 (Figure 1 C). Addition of 23A11 to cord blood-derived human CD34+ cells also enhanced the later stages of megakaryocyte proliferation (on day 14) as illustrated by flow cytometry using a FITC-conjugated CD41 antibody, whereas on day 5 no differences in CD41 expression were observed (Figure 2).

### EXAMPLE 5. Effect of VPAC1 receptor antibody on myelosuppressive therapy induced thrombocytopenia.

We administered 1 mg/kg 23A11 antibody reconstituted in 40 % polyethylene glycol 400 (PEG400) by subcutaneous injections on days 0, 3, and 7 for mice (n=5 for 23A11 and n=15 for PBS) and on days 0, 3, 7, and 10 for New Zealand White (NZW) rabbits (n=3 for 23A11 or n=3 for PBS). To induce myelosuppression, recipients were treated with a double dose of busulfan (Orphan Medical Inc., Minnetonka, MN, USA) (20 mg/kg intraperitoneally) on days 7 and 10. This dose of busulfan preferentially suppresses the megakaryocytic lineage in mice and rabbits (Kuter et al. 1995). At different time intervals, blood was removed for cell counts.

The GraphPad InStat 3.01 software (GraphPad Software Inc., San Diego, CA) was used for statistical analysis. Continuous variables with little to mild skewness were summarized as mean ± SD and compared by means of the Student t test for unpaired data. To determine the differences in platelet counts after myelosuppressive therapy, a one-way ANOVA test was used, complemented with Bonferroni's multiple comparison t-Test to identify statistically differences at each individual time point. We used GraphPad Prism4 software for the statistical analysis of survival times during myeloablative therapy with the Kaplan-Meier test followed by the Logrank test. Data were considered significant in all cases when the P value was less than
- 05.

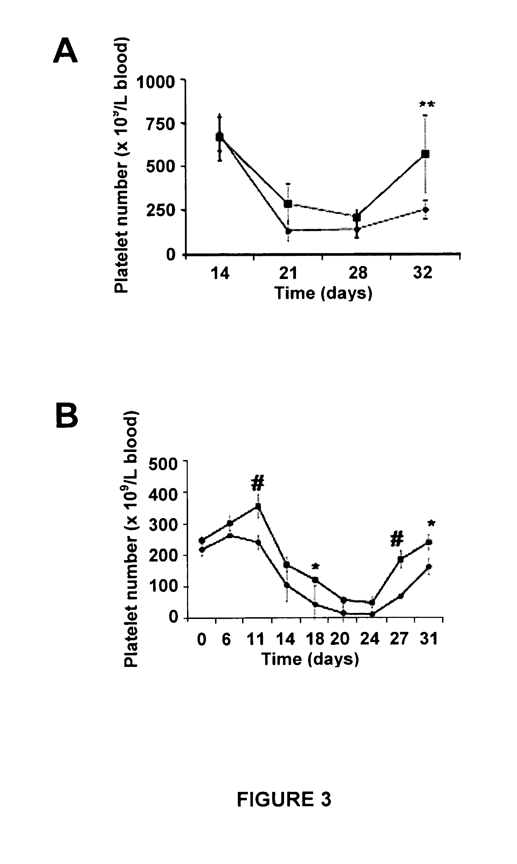

The subcutaneous injection of 23A11 (on days 0, 3 and 7) was capable of stimulating platelet counts in mice after chemotherapy-induced thrombocytopenia (busulfan treatment on days 7 and 10) (Figure 3A). In the group treated with PBS, 5 out of 15 mice died between 2 and 22 days after the last busulfan injection because of internal bleeding, whereas in the group treated with 23A11 all mice survived chemotherapy.

The effect of anti-VPAC1 receptor antibody 23A11 on in vitro megakaryocyte differentiation was more pronounced than that of anti-PACAP monoclonal antibody PP1A4 and therefore the in vivo study in a larger animal model was carried out with 23A11. The subcutaneous injection with 23A11 (on days 0, 3, 7, and 10) reduced the deep drop in platelet count and accelerated the recovery of the platelet number in busulfan treated rabbits (Figure 3B). Rabbits injected with 23A11 and treated with busulfan show increased platelet numbers (188 ± 27 x 10⁹ plt/L on day 27) compared to rabbits injected with PBS (72 ± 14 x 10⁹ plt/L on day 27, p < 0.001 by ANOVA). Except from a stimulatory role on the megakaryocytic lineage, we never observed any effect of the inhibition of VPAC1 signaling on the number of red blood cells or leukocytes.

### EXAMPLE 6. Effect of anti-VPAC1 receptor antibody on GATA1 deficiency-induced thrombocytopenia.

Isolated human CD34+ cord blood cells (1.5 x10⁵) were cultured in Iscove's modified Dulbecco medium (IMDM) with stable glutamine (Invitrogen Corp., Carlsbad, California, USA), which was supplemented with 0.5% bovine serum albumin, 200 µg/mL iron-saturated transferrin, 10 µg/ml human insulin, 50 µM β-mercaptoethanol (all from Sigma Chemical, St Louis, MO), 25 ng/ml thrombopoietin (TPO), 10 ng/ml IL6, 10 ng/ml IL3, 25 ng/ml stem cell factor (SCF) (all from Peprotech, London, UK). To determine megakaryocyte ploidy, bone marrow cells or in vitro differentiated megakaryocytes (day 12) were costained with FITC-conjugated CD41a or FITC-conjugated CD61and propidium iodide (Sigma), and the DNA content was determined by two-color flow cytometry (Mathur et al. 2004). Expanding CD41+ or mature CD61⁺ megakaryocytes were also quantified by flow cytometry on days 5, 12 and 14. We used Cell Quest software for two-color immunofluorescence acquisition on a FACSCalibur flow cytometer (BD Biosciences) and for data analysis.

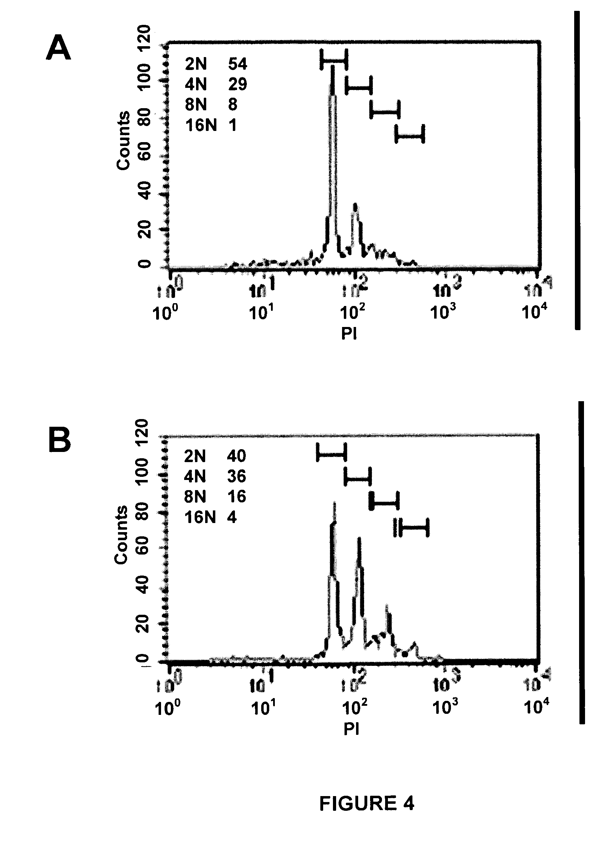

Isolated CD34+ cells from bone marrow of a GATA1 deficient patient with a severe thrombocytopenia (Freson et al. 2002) were differentiated in vitro into megakaryocytes, in the absence or presence of monoclonal antibody 23A11. FACS analysis at day 12 of the differentiated GATA1-D218Y deficient megakaryocytes showed a maturation defect with immature megakaryocytes (mostly 2N phase) while added 23A11 again increased the DNA ploidy of these megakaryocytes to 16N (Figure 4).

### EXAMPLE 7. Sequencing and humanization of murine antibodies.

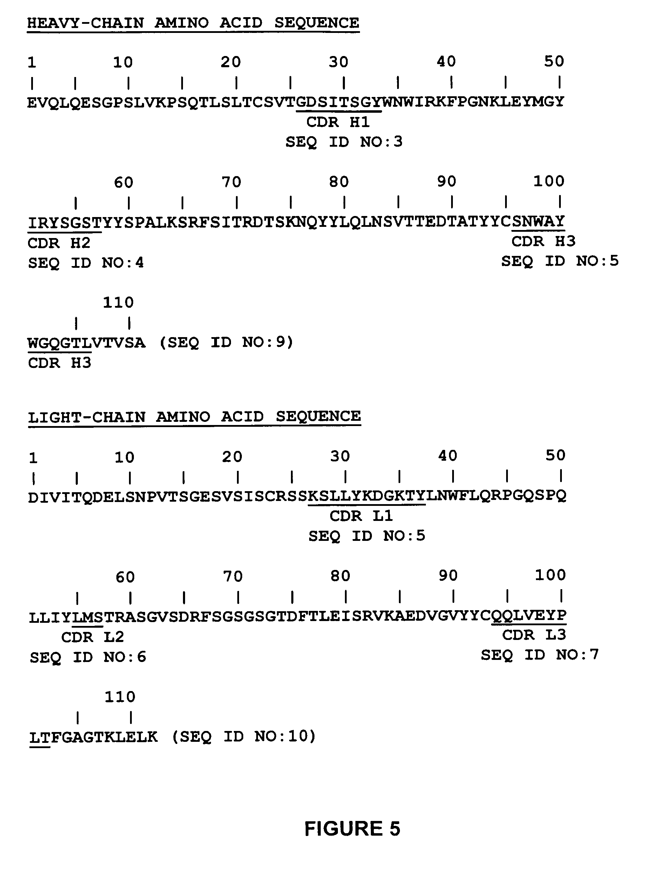

The variable regions of the murine antibody 23A11 obtained in Example 4 were cloned and sequenced basically as described in Example 1 of WO 2006/099698. From the nucleotide sequences were derived the light- and heavy-chain amino acid sequences (see Figure 5). The heavy-chain variable region is defined by SEQ ID NO:9 and comprises CDR H1, CDR H2, and CDR H3 defined by SEQ ID NOs: 3, 4, and 5, respectively. The light-chain variable region is defined by SEQ ID NO:10 and comprises CDR L1, CDR L2, and CDR L3 defined by SEQ ID NOs: 6, 7, and 8, respectively.

The hybridoma cell line expressing murine antibody 23A11 was deposited on June 19, 2007 under the conditions of the Budapest Treaty at the Belgian Coordinated Collections of Microorganisms (BCCM) LMBP Collection (BCCM-LMBP / Universiteit Gent / Technologiepark 927 / B-9052 Gent-Zwijnaarde /Belgium).The hybridoma cell line has been assigned accession number LMBP 6579CB.

The humanization of the murine antibody 23A11, as well as the expression or recombinant human (rhu) 23A11 antibodies, was performed basically as outlined in Example 6 of WO 2006/099698. The variable domains of the murine antibody 23A11 were grafted on human IgG1-type and IgG4-type antibody backbones (constant domains).

A number of amino acid substitutions were introduced in the humanized variable domains as compared to the murine variable domain. Sequences of the rhu23A11 IgG1 heavy- and light chains are defined by/represented in SEQ ID NOs: 11 and 12, respectively. Sequences of the rhu23A11 IgG4 heavy- and light chains are defined by/represented in SEQ ID NOs: 13 and 14, respectively. Over the whole variable regions, excluding the CDR sequences, the humanized light chain is 90 % identical to the murine light chain; for the humanized heavy chain this is 83,5%. In particular the following amino acid substitutions have been introduced:
- in murine heavy chain variable region (SEQ ID NO:9): Ser at position 10 to Gly; Ser at position 23 to Thr; Thr at position 25 to Ser; Phe at position 40 to His; Asn at position 43 to Lys; Lys at position 44 to Gly; Ala at position 62 to Ser; Ser at position 68 to Thr; Thr at position 70 to Ser; Tyr at position 79 to Ser; Gln at position 81 to Lys; Asn at position 83 to Ser; Thr at position 87 to Ala; and Glu at position 88 to Ala;
- in murine light chain variable region (SEQ ID NO:10): Asp at position 7 to Ser; Glu at position 8 to Pro; Asn at position 11 to Leu; Ser at position 15 to Pro; Ser at position 18 to Pro; Glu at position 79 to Lys; Lys at position 84 to Glu; Ala at position 105 to Gln; and Leu at position 111 to Ile;
- wherein the positions of the amino acids are relative to SEQ ID NOs:9 and 10, respectively.

### EXAMPLE 8. Quantification of binding of anti-VPAC1 antibodies to VPAC1.

For kinetic binding analysis of GST-VPAC1 with the GST-VPAC1-antibodies, we used a BIAcore surface plasmon resonance system at 25 °C. These interactions were performed on a BIAcore 1000. GST-VPAC1 was covalently immobilized on a CM5 sensorchip (carboxylated dextran matrix) after activation with a 1:1 mixture of 1-ethyl-3- (3-dimethylaminopropyl) carbodiimide and *N-*hydroxysuccinimide. The remaining active sites on the chip were blocked by 1.0 M ethanolamine-HCl at pH 8.5. To perform binding assays with the GST-VPAC1-antibodies, various concentrations (ranging from 250nm to 5nm) of murine 23A11 were injected over the immobilized GST-VPAC-1 at a flow rate of 30 µl/min. This was performed in a running buffer consisting of 0.01 M HEPES at pH 7.4, 0.15M NaCl, 3mM EDTA and 0.005% Surfactant P20 (or Tween 20). The sensorgrams were evaluated using the BlAevaluation 4.1 software. The rate constants kₐ and k_{d} and the dissociation constant K_{D} were determined by using a global fit with a Langmuir 1:1 binding model with baseline drift. Dissociation constants K_{D} of 2,83e-8 M and 1,99e-8 M were obtained for two different batches of murine 23A11 antibody. This corresponds to affinity constants K_{A} = 1/K_{D} of 3,54e+7 M⁻¹ and 5,04e+7 M⁻¹, respectively. A K_{D} value of 9,82e-9 M was obtained for a humanize 23A11 antibody, this corresponds to an affinity constant K_{A} of 1,02e+8 M⁻¹.

### EXAMPLE 9. Determination of VPAC1-epitopes

For the epitope mapping of 23A11, several VPAC1 deletion variants were fused to GST and expressed. Binding of 23A11 to the VPAC1 deletion variants was tested by western blotting.

The following VPAC1 variants were used:
- full length VPAC1 spanning amino acids 1-457 of SEQ ID NO:15;
- VPAC1 variant 4-2 spanning amino acids 231-457 of SEQ ID NO:15;
- VPAC1 variant 3-2 spanning amino acids 123-457 of SEQ ID NO:15; and
- VPAC1 variant 1-6 spanning amino acids 1-237 of SEQ ID NO:15.

VPAC1 variant 4-2 (and, to a lesser extent, variant 3-2) was recognized by 23A11. Since this protein contains the second as well as the third extracellular loop of VPAC1, both loops (extracellular loop 2: SEQ ID NO:1; extracellular loop 3:SEQ ID NO:1) were made as GST fusion proteins and tested in western blotting. The murine antibody 23A11 was binding to each of these loops.

### EXAMPLE 10. Anti-VPAC1 antibodies and PACAP are not competing for binding the VPAC1-receptor protein

Preliminary data from binding competition studies revealed that the murine anti-VPAC1 receptor antibody 23A11 seemed not to be inhibiting binding of PACAP to the VPAC1 receptor.

### CITED REFERENCES

1. Barton, G. M., and Medzhitov, R. Toll-like receptors and their ligands. Curr. Top. Microbiol. Immunol. 270:81-92, 2002.
2. Branch, D. R., Valenta, L. J., Yousefi, S., Sakac, D., Singla, R., Bali, M., Sahai, B. M., and Ma, X. Z. VPAC1 is a cellular neuroendocrine receptor expressed on T cells that actively facilitates productive HIV-1 infection. AIDS 16:309-319, 2002.
3. Bussel, J. B., Kuter, D. J., George, J. N., McMillan, R., Aledort, L. M., Conklin, G. T., Lichtin, A. E., Lyons, R. M., Nieva, J., Wasser, J. S., Wiznitzer, I., Kelly, R., Chen, C. F., and Nichol, J. L. AMG 531, a thrombopoiesis-stimulating protein, for chronic ITP. N. Engl. J. Med. 355:1672-1681, 2006.
4. Busto, R., Prieto, J. C., Bodega, G., Zapatero, J., and Carrero, I. Immunohistochemical localization and distribution of VIP/PACAP receptors in human lung. Peptides 21:265-269, 2000.
5. Byl, B., Libin, M., Bauer, J., Martin, O. R., De, W. D., Davies, G., Goldman, M., and Willems, F. OM197-MP-AC induces the maturation of human dendritic cells and promotes a primary T cell response. Int. Immunopharmacol. 3:417-425, 2003.
6. Fahrenkrug, J., Hannibal, J., Tams, J., and Georg, B. Immunohistochemical localization of the VIP1 receptor (VPAC1 R) in rat cerebral blood vessels: relation to PACAP and VIP containing nerves. J. Cereb. Blood Flow Metab 20:1205-1214,2000.
7. Freson, K., Matthijs, G., Thys, C., Marien, P., Hoylaerts, M. F., Vermylen, J., and Van, G. C. Different substitutions at residue D218 of the X-linked transcription factor GATA1 lead to altered clinical severity of macrothrombocytopenia and anemia and are associated with variable skewed X inactivation. Hum. Mol. Genet. 11:147-152, 2002.
8. Freson, K., Jaeken, J., Van, H. M., de, Z. F., Wittevrongel, C., Thys, C., Hoylaerts, M. F., Vermylen, J., and Van, G. C. Functional polymorphisms in the paternally expressed XLalphas and its cofactor ALEX decrease their mutual interaction and enhance receptor-mediated cAMP formation. Hum. Mol. Genet. 12:1121-1130, 2003.
9. Freson, K., Hashimoto, H., Thys, C., Wittevrongel, C., Danloy, S., Morita, Y., Shintani, N., Tomiyama, Y., Vermylen, J., Hoylaerts, M. F., Baba, A., and Van, G. C. The pituitary adenylate cyclase-activating polypeptide is a physiological inhibitor of platelet activation. J. Clin. Invest 113:905-912, 2004.
10. Goetzl, E. J., Patel, D. R., Kishiyama, J. L., Smoll, A. C., Turck, C. W., Law, N. M., Rosenzweig, S. A., and Sreedharan, S. P. Specific recognition of the human neuroendocrine receptor for vasoactive intestinal peptide by antipeptide antibodies. Mol. Cell Neurosci. 5:145-152, 1994.
11. Johnson, D. A., Sowell, C. G., Johnson, C. L., Livesay, M. T., Keegan, D. S., Rhodes, M. J., Ulrich, J. T., Ward, J. R., Cantrell, J. L., and Brookshire, V. G. Synthesis and biological evaluation of a new class of vaccine adjuvants: aminoalkyl glucosaminide 4-phosphates (AGPs). Bioorg. Med. Chem. Lett. 9:2273-2278, 1999.
12. Kabat, E. A., Wu, T. T., Perry, H. M., and Gottesman, K. S. F. C. Sequences of proteins of immunological interest, 5th edition. 1991. U.S. Department of Health and Human Services, Bethesda, MD.
13. Kaushansky, K. Thrombopoietin. N. Engl. J. Med. 339:746-754, 1998.
14. Kuter, D., Bussel, J., George, J., and et al. Long-term dosing of AMG 531 in thrombocytopenic patients with immune thrombocytopenic purpura: 48-week update. Blood 108:168, 2006.
15. Kuter, D. J., and Rosenberg, R. D. The reciprocal relationship of thrombopoietin (c-Mpl ligand) to changes in the platelet mass during busulfan-induced thrombocytopenia in the rabbit. Blood 85:2720-2730, 1995.
16. Langlet, C., Langer, I., Vertongen, P., Gaspard, N., Vanderwinden, J. M., and Robberecht, P. Contribution of the carboxyl terminus of the VPAC1 receptor to agonist-induced receptor phosphorylation, internalization, and recycling. J. Biol. Chem. 280:28034-28043, 2005.
17. Mathur, A., Hong, Y., Wang, G., and Erusalimsky, J. D. Assays of megakaryocyte development: surface antigen expression, ploidy, and size. Methods Mol. Biol. 272:309-322, 2004.
18. Persing, D. H., Coler, R. N., Lacy, M. J., Johnson, D. A., Baldridge, J. R., Hershberg, R. M., and Reed, S. G. Taking toll: lipid A mimetics as adjuvants and immunomodulators. Trends Microbiol. 10:S32-S37, 2002.
19. Riedl, P., Buschle, M., Reimann, J., and Schirmbeck, R. Binding immune-stimulating oligonucleotides to cationic peptides from viral core antigen enhances their potency as adjuvants. Eur. J. Immunol. 32:1709-1716, 2002.
20. Schulz, S., Rocken, C., Mawrin, C., Weise, W., Hollt, V., and Schulz, S. Immunocytochemical identification of VPAC1, VPAC2, and PAC1 receptors in normal and neoplastic human tissues with subtype-specific antibodies. Clin. Cancer Res. 10:8235-8242, 2004.
21. Shreeve, M. S. Identification of G-proteins coupling to the vasoactive intestinal peptide receptor VPAC(1) using immunoaffinity chromatography: evidence for precoupling. Biochem. Biophys. Res. Commun. 290:1300-1307, 2002.
22. Vaudry, D., Gonzalez, B. J., Basille, M., Yon, L., Fournier, A., and Vaudry, H. Pituitary adenylate cyclase-activating polypeptide and its receptors: from structure to functions. Pharmacol. Rev. 52:269-324, 2000.
23. Ware, R. E., and Zimmerman, S. A. Anti-D: mechanisms of action. Semin. Hematol. 35 (Suppl. 1):14-22, 1998.
24. Winter, G., and Harris, W. J. Humanized antibodies. Immunol. Today 14:243-246, 1993.

## Claims

1. A monoclonal antibody to the VPAC1 receptor wherein said antibody is specifically binding to an extracellular domain of said VPAC1 receptor, or a functionally equivalent fragment of said antibody.

2. The antibody or fragment thereof according to claim 1 wherein said antibody is specifically binding to extracellular loop 2 and/or to extracellular loop 3 of said VPAC1 receptor.

3. The antibody or fragment thereof according to claim 1 or 2 which is, upon binding *in vitro* to the VPAC1 receptor on VPAC1 receptor-carrying cells, decreasing cAMP levels in said cells.

4. The antibody or fragment thereof according to claim 1 or 2 which is enhancing maturation of *in vitro* cultured immature megakaryocyte cells.

5. The antibody or fragment thereof according to any one of claims 1 to 4 which is binding to the VPAC1 receptor extracellular loop 2 epitope comprised in SEQ ID NO:1, binding to a protein comprising said epitope, or binding to a protein comprising SEQ ID NO:1.

6. The antibody or fragment thereof according to any one of claims 1 to 4 which is binding to the VPAC1 receptor extracellular loop 3 epitope comprised in SEQ ID NO:2, binding to a protein comprising said epitope, or binding to a protein comprising SEQ ID NO:2.

7. The antibody or fragment thereof according to any one of claims 1 to 4 which is binding both to (i) to the VPAC1 receptor extracellular loop 2 epitope comprised in SEQ ID NO:1, binding to a protein comprising said epitope, or binding to a protein comprising SEQ ID NO:1, and to (ii) the VPAC1 receptor extracellular loop 3 epitope comprised in SEQ ID NO:2, binding to a protein comprising said epitope, or binding to a protein comprising SEQ ID NO:2.

8. The antibody or fragment thereof according to any one of claims 1 to 7 which is **characterized by** comprising at least one of the CDR amino acid sequences chosen from SEQ ID NOs: 3-8 or at least one of said CDR amino acid sequences wherein 1 amino acid is changed.

9. The antibody or fragment thereof according to any one of claims 1 to 7 which is **characterized by** comprising at least the heavy-chain variable region defined by SEQ ID NO:9, or by comprising a heavy-chain variable region having at least 83,5% identity to SEQ ID NO:9 outside the CDRs, and wherein the CDR amino acid sequences are defined by SEQ ID NOs: 3-5, by said CDR amino acid sequences wherein 1 amino acid is changed, or by any combination of changed and unchanged CDRs.

10. The antibody or fragment thereof according to any one of claims 1 to 7 which is **characterized by** comprising at least the light-chain variable region defined by SEQ ID NO:10, or by comprising a light-chain variable region having at least 90% identity to SEQ ID NO:10 outside the CDRs, and wherein the CDR amino acid sequences are defined by SEQ ID NOs:6-8, by said CDR amino acid sequences wherein 1 amino acid is changed, or by any combination of changed and unchanged CDRs.

11. A murine monoclonal antibody which is produced by a hybridoma cell line with biological deposit accession number LMBP 6579CB; or a functionally equivalent fragment of said antibody.

12. A monoclonal antibody to the VPAC1 receptor which is specifically binding to said VPAC1 receptor as bound by the antibody according to any one of claims 1 to 11, or a functionally equivalent fragment of said monoclonal antibody.

13. The antibody according to claim 12 which is a humanized monoclonal antibody, or a functionally equivalent fragment thereof.

14. The antibody according to claim 13 having a human IgG1-type heavy chain with the sequence defined by SEQ ID NO:11, or a functionally equivalent fragment thereof.

15. The antibody according to claim 13 having a human IgG1-type light chain with the sequence defined by SEQ ID NO:12, or a functionally equivalent fragment thereof.

16. The antibody according to claim 13 having a human IgG4-type heavy chain with the sequence defined by SEQ ID NO:13, or a functionally equivalent fragment thereof.

17. The antibody according to claim 13 having a human IgG4-type light chain with the sequence defined by SEQ ID NO:14, or a functionally equivalent fragment thereof.

18. The antibody according to any one of claims 1 to 17 which is binding to the VPAC1 receptor defined in SEQ ID NO:15 with an affinity constant of at least 1e+8 M⁻¹, or a functionally equivalent fragment of said antibody.

19. The antibody according to any one of claims 1 to 18 wherein said functionally equivalent fragment thereof is a Fab, Fab', F(ab')2, scFv fragment or nanobody.

20. A functional derivative of the antibody or fragment thereof according to any one of claims 1 to 19.

21. A composition comprising the antibody or fragment thereof according to any one of claims 1 to 19, or comprising the functional derivative according to claim 20, and at least one of a diluent, carrier or adjuvant.

22. The antibody or fragment thereof according to any one of claims 1 to 19, or the functional derivative according to claim 20, for use as medicament.

23. A hybridoma cell line expressing an antibody or fragment thereof according to any one of claims 1 to 19.

24. The hybridoma cell line according to claim 23 with biological deposit accession number LMBP 6579CB.

25. An isolated nucleic acid sequence encoding the antibody or fragment thereof according to any one of claims 1 to 19.

26. A recombinant vector comprising the nucleic acid sequence according to claim 25.

27. The recombinant vector according to claim 26 which is an expression vector.

28. A recombinant host cell expressing an antibody or fragment thereof according to any one of claims 1 to 19.

29. A method of producing the antibody or fragment thereof according to any one of claims 1 to 19 comprising the steps of:
(i) obtaining a crude preparation of said antibody or antibody fragment by means of recombinant expression of said antibody or antibody fragment, or by means of chemical synthesis of said antibody or antibody fragment; and
(ii) purifying said antibody or antibody fragment from the crude preparation obtained in (i).

30. A method of producing the fragment of the antibody according to any one of claims 1 to 19 comprising the steps of:
(i) obtaining a crude preparation of an antibody comprising said fragment by means of recombinant expression of said antibody or by means of chemical synthesis of said antibody ;
(ii) purifying said antibody from the crude preparation obtained in (i); and
(iii) isolating said fragment from the antibody purified in (ii).

31. Use of an antibody binding to the VPAC1 receptor or of a functionally equivalent fragment of said antibody, or of a composition comprising any thereof, for the preparation of a medicament to stimulate maturation of immature megakaryocytes in a mammal.

32. Use of an antibody binding to the VPAC1 receptor or of a functionally equivalent fragment of said antibody, or of a composition comprising any thereof, for the preparation of a medicament to treat a thrombocytopenic mammal.

33. Use of an antibody binding to the VPAC1 receptor or of a functionally equivalent fragment of said antibody, or of a composition comprising any thereof, for the preparation of a medicament to reduce thrombocytopenia in a mammal subjected to thrombocytopenia-inducing conditions.

34. The use according to any one of claims 31 to 33 wherein said medicament is administered prior to, concomitant with or after administration of a further compound.

35. The use according to claim 34 wherein said further compound is chosen from thrombopoietin, AMG 531, or Interleukin-11.

36. The use according to any one of claims 31 to 35 wherein said antibody or fragment thereof is binding to an extracellular domain of the VPAC1 receptor.

37. The use according to claim 36 wherein said extracellular domain is an extracellular loop of the VPAC1 receptor.

38. The use according to claim 36 wherein said extracellular domain is the extracellular loop 2 and/or extracellular loop 3 of the VPAC1 receptor.

39. The use according to any one of claims 31 to 38 wherein said antibody or fragment is a monoclonal antibody or a functionally equivalent fragment thereof.

40. The use according to any one of claims 31 to 39 wherein said mammal is a human.

41. The use according to any one of claims 31 to 40 wherein said antibody binding to the VPAC1 receptor is the antibody according to any one of claims 1 to 19.
